# EUROPEAN PATENT APPLICATION

(11) **EP 4 509 528 A1**
(43) Date of publication of application: **19.02.2025**
(21) Application number: 24195062.5
(22) Date of filing: 16.08.2024
(51) Int. Cl.: C07K 16/26

(54) **MONOCLONAL ANTIBODIES AGAINST VETERINARY PROGASTRIN-LIKE MOLECULES AND THEIR USES**

(30) Priority: 16.08.2023 US 202363532965 P
(71) Applicant: Barrelman Biosciences LLC, Los Alamitos, CA 90720 (US)
(72) Inventor: COHEN, William, Los Alamitos, 90720 (US); RAY, Robert, Los Alamitos, 90720 (US)
(74) Representative: Barker Brettell LLP

(57) **Abstract**

Described herein are veterinary progastrin binding molecules (VPBM) against veterinary progastrin-like molecules (VPM) and the methods of making and using the VPBM thereof. Also described, are compositions comprising binding domains of VPBM against VPM and methods of making an using thereof.

## Description

### CROSS-REFERENCE TO RELATED APPLICATIONS

This application claims priority to and incorporates by reference U.S. Provisional Application Serial Number 63/532,965 filed August 16, 2023, the contents of which are incorporated herein by reference.

### TECHNICAL FIELD

Monoclonal antibodies against and binding domains against veterinary progastrin-like molecules (VPM) and their uses.

### BACKGROUND

Various antibodies against veterinary progastrin-like molecules (VPM) have been proposed. Because these progastrin binding molecules (PBM) are not optimal for veterinary sources, there is a need in the field for improved veterinary progastrin binding molecules (VPBM), including monoclonal antibodies against VPM.

### BRIEF DESCRIPTION OF THE DRAWINGS

Fig. 1 shows a schematic diagram of an exemplary vector for cloning the heavy chain of a monoclonal antibody against a VPM.
Fig. 2 show a schematic diagram of an exemplary vector for cloning the light chain of a monoclonal antibody against a VPM.
Fig. 3 show Canine recombinant progastrin antibodies (1-H2 and 2-A3) did not bind to the human recombinant progastrin.
Fig. 4 show canine recombinant progastrin antibodies (1-H2 and 2-A3) did not bind to canine gastrin (standards from LS Bio's ELISA kit).

### DETAILED DESCRIPTION

### I. Interpretations and Definitions

Unless otherwise indicated, this description employs conventional chemical, biochemical, molecular biology, immunology, and pharmacology methods and terms that have their ordinary meaning to persons of skill in this field. All publications, references, patents, and patent applications cited herein are hereby incorporated by reference in their entireties.

As used in this specification and the appended claims, the following general rules apply.

Singular forms "a," "an," and "the" include plural references unless the content clearly indicates otherwise.

The term "about" takes on its plain and ordinary meaning of "approximately" as a person of skill in the art would understand. When the term "about" is used in conjunction with a numerical range, it modifies that range by extending the boundaries above and below the numerical values set forth. In general, the term "about" or "approximately" is used herein to modify a numerical value above and below the stated value by a variance of 20%.

As used herein, the recitation of a numerical range for a variable is intended to convey that the invention may be practiced with the variable equal to any of the values within that range. Thus, for a variable which is inherently discrete, the variable can be equal to any integer value of the numerical range, including the end-points of the range. Similarly, for a variable which is inherently continuous, the variable can be equal to any real value of the numerical range, including the end-points of the range. As an example, a variable which is described as having values between 0 and 10, can be 0, or 1, or 2, or 3..., or 10 for variables which are inherently discrete, and can be 0.0, 0.1, 0.01, 0.001, or any other real value for variables which are inherently continuous.

As used herein, the following terms shall have the specified meaning.

A "veterinary progastrin binding molecule" or "VPBM" means a molecule that binds to a VPM and has low binding affinity for human progastrin. Preferably, the binding affinity for human progastrin is less than about 50% of the binding affinity for a veterinary progastrin. More preferably, the binding affinity for a human progastrin is less than about 10% of the binding affinity for a veterinary progastrin. More preferably, the binding affinity for human progastrin is less than about 1% of the binding affinity for a veterinary progastrin. More preferably, the binding affinity for human progastrin is less than about 0.1% of the binding affinity for a veterinary progastrin. More preferably, the binding affinity for human progastrin is less than about 0.01% of the binding affinity for a veterinary progastrin. More preferably, the binding affinity for human progastrin is less than about 0.001% of the binding affinity for a veterinary progastrin. More preferably, the binding affinity for human progastrin is less than about 0.0001% of the binding affinity for a veterinary progastrin. More preferably, the binding affinity for human progastrin is less than about 0.00001% of the binding affinity for a veterinary progastrin. More preferably, the binding affinity for human progastrin is less than about 0.000001% of the binding affinity for a veterinary progastrin. More preferably, the binding affinity for human progastrin is less than about 0.0000001% of the binding affinity for a veterinary progastrin. Preferably, the binding affinity for human progastrin is equivalent to the binding affinity for BSA and/or skim milk powder. Examples of VPBM include a monoclonal antibody against a VPM, a binding domain of monoclonal antibody against a VPM, and the likes thereof. A VPBM can be a monoclonal antibody (multiple chain or single chain antibodies), fragments of a monoclonal antibody that selectively bind VPB (e.g., Fab, Fab', (Fab')2, Fv, and scFv), surrobodies (e.g. surrogate light chain construct), single domain antibodies, chimeric antibodies, humanized antibodies, camelized antibodies, and the like. In certain embodiments, the VPBM is selected from a suitable antibody isotype, or is derived from a suitable antibody isotype. Exemplary isotypes include IgA (e.g., IgA1 and IgA2), IgD, IgE, IgG (e.g., IgG1, IgG2, IgG3, and IgG4), and IgM. In certain embodiments, the VPBM is an IgG selected from IgG1, IgG2, IgG3, IgG4, and combinations thereof. VPBM can be originated from, for example, monoclonal antibodies from rabbits, rodents, goats, and other antibody producing organisms. VPBM can be a neutralizing, a non-neutralizing antibody, and/or a binding domain. Exemplary neutralizing VPBM is capable of yielding a statistically significant favorable change in veterinary tumor progression and veterinary cancer progression. Exemplary non-neutralizing (as well as neutralizing) monoclonal antibody and/or binding domain are suitable for use in veterinary diagnostics and/or prognosis. Embodiments of the VPBM can be a be formulated for veterinary use, such as veterinary diagnostic, prognostic, therapy, treatments, and combinations thereof.

"Neutralizing," when directed to a VPBM, means that the VPBM is capable of modulating and/or interfering with the function of progastrin and/or gastrin, including its signaling function. The neutralizing VPBM is capable of yielding a statistically significant reduction in a GPD/C, such as veterinary tumor progression and veterinary cancer progression, as compared to a non-specific monoclonal antibody. In general, a desirable neutralizing VPBM can exhibit at least about 50% reduction in the number of tumor and/or cancer cells, as compared to a control (i.e. a non-specific monoclonal antibody). A neutralizing antibody exhibiting a statistically significant reduction of about 10%, 15%, 20%, 30%, and/or 40% can also provide veterinary therapeutic effects to a GPD/C.

A "veterinary progastrin-like molecule" or "VPM" is a progastrin molecule that is not from a human source and does not have either SEQ ID NO: 5 or SEQ ID NO: 6. Examples of VPM can include amino acid residues essentially the same as SEQ ID NO: 1-4 and 28-99. "Essentially the same as" as used herein means a molecule having the same amino acid residues or having one (1) amino acid difference. An exemplary recombinant VPM is a veterinary progastrin-His SUMO protein. A VPM that elicits the production of antibodies is referred herein as a "veterinary progastrin-like immunogen."

A "gastrin-promoted disease or condition" ("GPD/C") means any disease or condition in which gastrin and/or progastrin has a stimulatory or inhibitory role. A GPD/C can include gastrin-promoted tumor formation, gastric cancer, colorectal cancer, pancreatic cancer, small cell lung cancer, liver metastasized tumor, gastritis, gastric-intestinal tumor, thyroid cancer, and prostate cancer.

"Veterinary" means of, relating to, practicing, or being the science and art of prevention, cure, or alleviation of disease and injury in animals, including domestic animals.

"Veterinary subject" means a non-human subject. In certain embodiments, the veterinary subject is a mammal in the orders of Carnivora, Rodentia, Chiroptera, Artiodactyla, Perissodactyla, Cetacea, and Eulipotyphla. In certain embodiments, the veterinary subject can be Canis lupus familiaris, Vulpes vulpes, Vulpes lagopus, Phyllostomus discolor, Phyllostomus hastatus, Erinaceus europaeus, Felis catus, Panthera tigris, Lynx canadensis, Equus caballus, Equus asinus, Ailuropoda melanoleuca, Myotis davidii, Myotis brandtii, Myotis lucifugus, Eptesicus fuscus, Leopardus geoffroyi, Pipistrellus kuhlii, Panthera leo, Nyctereutes procyonoides, Lipotes vexillifer, Pteronotus parnellii mesoamericanus, Mus musculus, Mus caroli, Acomys russatus, Mesocricetus auratus, Microtus fortis, Cricetulus griseus, Mus pahari, Myodes glareolus, Microtus oregoni, Arvicola amphibius, Phodopus roborovskii, Onychomys torridus, Peromyscus maniculatus bairdii, Peromyscus californicus insignis, Mastomys coucha, Arvicanthis niloticus, Myotis myotis, Castor canadensis, Hyaena hyaena, Galemys pyrenaicus, Neophocaena asiaeorientalis asiaeorientalis, Delphinapterus leucas, Monodon monoceros, Phocoena sinus, Sousa chinensis, Lagenorhynchus obliquidens, Orcinus orca, Bos taurus, Ursus maritimus, Ursus americanus, Ovis ammon polii x Ovis aries, Cervus elaphus hippelaphus, Ovis aries, Budorcas taxicolor, Peromyscus leucopus, Marmota monax, Sciurus carolinensis, Marmota marmota marmota, Marmota flaviventris, Grammomys surdaster, Ictidomys tridecemlineatus, Miniopterus natalensis, Molossus molossus, Nannospalax galili, Rattus norvegicus, Rattus rattus, Ochotona princeps, Mirounga leonina, Halichoerus grypus, Leptonychotes weddellii, Mirounga angustirostris, Odobenus rosmarus divergens, Eumetopias jubatus, Callorhinus ursinus, Zalophus californianus, Lontra canadensis, Mustela erminea, Lemur catta, Manis javanica, Diceros bicornis minor, Ceratotherium simum simum, Canis lupus familiaris, Marmota monax, Nyctereutes procyonoides, Urocitellus parryii, Sus scrofa, Phacochoerus africanus, Phoca vitulina, Neogale vison, Mustela erminea, Lutra lutra, Enhydra lutris kenyoni, Meles meles, Mustela putorius furo, Tupaia chinensis, Propithecus coquereli, Microcebus murinus, Galeopterus variegatus, Manis pentadactyla, Manis javanica, Macaca nemestrina, Papio anubis, Cercocebus atys, Mandrillus leucophaeus, Macaca mulatta, Macaca fascicularis, Theropithecus gelada, Echinops telfairi, Otolemur gamettii, Orycteropus afer afer, Bubalus bubalis, Carlito syrichta, Pteronotus parnellii mesoamericanus, Vicugna pacos, Moschus berezovskii, Cervus canadensis, Odocoileus virginianus texanus, Bos taurus, Cervus elaphus, Oryx dammah, Muntiacus muntjak, Ovis aries, Muntiacus reevesi, Bos mutus, Capra hircus, Rangifer tarandus platyrhyncus, Cervus elaphus hippelaphus, Ovis ammon polii x Ovis aries, Gulo gulo, Felis catus, Lynx canadensis, Dasypus novemcinctus, Camelus ferus, Myotis myotis, Dipodomys spectabilis, Eschrichtius robustus, and combinations thereof. In certain embodiments, the veterinary subject is selected from Table 12-Table 47. In certain embodiments, the veterinary subject is selected from Table 12-Table 25, and combinations thereof. In certain embodiments, the veterinary subject is selected from Table 26-Table 47, and combinations thereof. In certain embodiments, the veterinary subject is selected from Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain embodiments, the veterinary subject is selected from Table 12, Table 15, Table 16, Table 26, Table 30, Table 31, Table 32, Table 34, Table 40, and combinations thereof. In certain embodiments, the veterinary subject is selected from a mammal such as a canine (e.g., a dog), a feline (e.g., a cat, a tiger, and a lion), a bear (e.g. a panda and a polar bear), an equine (e.g. a horse and a donkey), a rodent (e.g., a mouse, a hamster, and a squirrel), a swine (e.g., a pig), and a deer, a goat, a sheep, a cattle, and combinations thereof. In certain embodiments, the veterinary subject is selected from a non-human primate such as a macaque (e.g., a rhesus macaque and a lemur). In certain embodiments, the veterinary subject is selected from domestic veterinary sources such as a domestic dog, a domestic cat, a domestic horse, a domestic donkey, a domestic pig, a domestic deer, a domestic goat, a domestic sheep, a domestic cattle, and combinations thereof. In certain embodiments, the veterinary subject is selected from one or more laboratory non-human primate models such as a macaque (e.g., a rhesus macaque), a lemur, and combinations thereof. In certain embodiments, the veterinary subject is a domestic dog. In certain embodiments, the veterinary subject is a domestic cat. In certain embodiments, the veterinary subject is a domestic horse.

A "bodily fluid" means a fluid from a body of a mammal. Exemplary body fluids include blood, plasma, serum, lymph, cerebrospinal fluid, saliva, sweat, and urine.

"Treating" or "treat" means reducing or alleviating symptoms in a subject to which the compound, composition, and/or method as described herein has been administered, as compared to the symptoms of a subject not being treated as described herein. The expression "slowing the progression of" means "hindering or inhibiting the development of". A practitioner will appreciate that the compounds, compositions, dosage forms, and methods described herein are to be used in concomitance with continuous clinical evaluations by a skilled practitioner (physician or veterinarian) to determine subsequent therapy. Such evaluation will aid and inform in evaluating whether to increase, reduce or continue a particular treatment dose, and/or to alter the frequency of administration.

A "preservative" means an agent, supplement or additive added to a composition that reduces the time-dependent degradation of a biological molecule in the composition. A pharmacologically acceptable non-toxic preservative means a preservative, when administered to a veterinary subject, at the prescribed concentration will cause negligible toxicity to the veterinary subject. Exemplary preservatives include sodium azide, EDTA and protease inhibitors, such as PMSF (Phenylmethylsulfonylfluoride) and aprotinin (e.g., Trasylol); citric acid; salt; a biological preservative, such as heparin; and combinations thereof.

An "excipient" means an agent and inactive ingredients that is added to a therapeutic formulation to stabilize and maintain a desirable pH, among others. Exemplary excipient includes a buffers (e.g., phosphate-buffered saline (PBS), histidine, citrate, and acetate, among others); tonicity agents (e.g., sodium chloride and mannitol, among others); stabilizers (e.g., sucrose, trehalose, glycerol, propylene glycol, arginine, and proline, among others); surfactants (e.g., polysorbate 20, polysorbate 80, and poloxamer 188, among others); chelating agents (e.g., ethylenediaminetetraacetic acid (EDTA), among others); antioxidants (e.g., methionine and ascorbic, among others); preservatives (e.g., benzyl alcohol and M-cresol, among others); bulking agents (e.g., mannitol and sucrose, among others); and freeze-drying (or lyophilization) protectants (e.g., dextran and polyethylene glycol (PEG), among others).

A route for "administration" means administering the therapeutic orally, transdermally, subcutaneously, intranasally, intramuscularly, intraocularly, topically, intrathecally, intracerebroventricular, intravenously, subcutaneously, intrathecal/intraventricularly, intravitreally, and/or intratumorally.

A "adverse effect" means an undesired or harmful effect resulting from a administering of a VPBM, where the negative outcome was not intended for. Exemplary adverse effects include allergic reactions, such as urticaria (hives), rash, pruritus (itching), swelling, difficulty breathing, and anaphylaxis, among others; gastrointestinal disturbances, such as vomiting, diarrhea, decreased appetite, and abdominal pain, among others; lethargy; weakness; restlessness; discomfort; fever; coughing; nasal discharge; increased respiratory rate or effort; rapid breathing; panting; tremors; seizures; behavior changes; anemia; thrombocytopenia (low platelet count); neutropenia (low neutrophil count); kidney or liver function changes; edema; blurred vision; among others.

A "recombinant" molecule means a molecule that is produced through recombinant DNA technology, by inserting the genes encoding the desired VPBM into a suitable expression system, where they are then transcribed and translated to produce the antibody.

A "diagnose" means determining whether a subject has a disease or condition.

A "prognosis" means the likelihood of a subject having a disease or condition. It can also mean the expected course or outcome of a disease or condition, including, for example, the likelihood of recovery, recurrence, and/or complications).

### II. Human and Veterinary progastrin molecules

Progastrin is a gastrin hormone precursor found in animals involved in not only gastric acid production but also proliferation, differentiation, and maturation of gastrointestinal cells. Precise detection and quantification of progastrin is thought to be important in the detection, diagnosis, and monitoring of gastrin-promoted diseases and conditions ("GPD/C"), including gastrin-promoted tumor formation, gastric cancer, colorectal cancer, pancreatic cancer, small cell lung cancer, liver metastasized tumor, gastritis, gastric-intestinal tumor, thyroid cancer and prostate cancer. In some gastrin-promoted diseases, progastrin can be detected in bodily fluids, such as blood, plasma, serum, and urine. There are also some interest in using human progastrin as a clinical marker for human disease progression.

In humans, a preprogastrin is produced by expression translation of the gastrin gene by the cells of the gastrointestinal track. An 80 amino acid human progastrin (hProGastrin) is then formed by cleaving the signal peptide in the first 21 amino acids at the N terminus from the 101 amino acid preprogastrin. The human progastrin is then processed by human enzymes to form various gastrin proteins to be secreted by gastric cells in response to gastrin-releasing peptide. In humans, progastrin is suppressed by gastric acid and pancreatic hormones, including somatostatin.

In humans, progastrin is also produced by tumor and/or cancerous cells, including colorectal tumor cells. It is believed that the production of progastrin encourages cell proliferation by moderating signaling transduction pathway to block cell differentiation and apoptosis. Some have proposed to treat human conditions with anti-gastrin and anti-human progastrin antibodies, to potentially slow tumor progression and promote apoptosis. However, diagnostic, prognostics, and treatments GPD/C in the veterinary field is not well developed.

### III. Veterinary progastrin are antigenically different from human progastrin molecules

Human progastrin and many veterinary progastrin have different protein sequences and structures. For example, human progastrin and veterinary progastrin have different epitopes. Moreover, antibody-like molecules raised against a human progastrin often lack the specificity to accurately detect veterinary progastrin from a specific species and/or veterinary progastrin in general. Thus, a challenge in the field of veterinary science is to develop a veterinary progastrin binding molecule (VPBM) specific for a veterinary species and/or veterinary progastrin in general. Moreover, because both human and veterinary species can produce progastrin, a further challenge would be to develop a VPBM with low affinity to human progastrin. Such a VPBM would reduce the risks of adverse effect to the human in the event that the veterinary therapeutic for the veterinary species (including veterinary companion species, such as dogs, cats, and horses) was accidentally consumed by human. The VPBM would also reduce the risk of human contamination in a veterinary diagnostics or prognostics. It would be even more challenging to develop a VPBM to detect veterinary progastrin across multiple species. Such a VPBM would reduce production overhead and logistics, and would provide benefits to not one, but a wide range of veterinary species. However, developing such a VPBM would be challenging and unpredictable. First, it has been challenging to identify a common immunogenic epitope shared by the veterinary species. Additionally, unspecific binding, which can reduce the accuracy of the diagnostics needs to be minimized..

### IV. Challenges for developing novel VMBP

While some have raised monoclonal antibodies to the N-terminus and C-terminus of human progastrin. However, monoclonal antibodies capable of selectively and accurately detect veterinary progastrin are not yet available. In particular, monoclonal antibodies capable of selectively and accurately detect veterinary progastrin, but not human progastrin, are not available. Likewise, monoclonal antibodies capable of sensitively and accurately detecting multiple veterinary progastrin are not yet available. Applicant is also not aware of any tests capable of quickly and accurately detecting traces amount of progastrin from veterinary bodily fluids.

It is challenging to raise monoclonal antibodies veterinary sources when the vast majority of the studies are directed towards human applications. When developing monoclonal antibodies, the selection of antigen is crucial to the utility of the antibody raised. It is also generally understood that not all antigens derived from progastrin peptides can produce strongly specific monoclonal antibodies against progastrin in physiological conditions. Thus, monoclonal antibodies raised from a human progastrin may not achieve the binding affinity and specificity needed for veterinary diagnostics, prognosis, and treatments.

Applicant has developed multiple VPBM; systems containing one or more VPBMs; nucleotide sequences and vector systems encoding the all or portions of the VPBM and systems containing VPBMs; hybridomas for the production of VPBM; methods of making the VPBM; and methods for using the VPBM for veterinary diagnostics, prognostics and treatment of a GPD/C. These VPBM have wide veterinary uses that are superior to monoclonal antibodies raised from human progastrin, because the VPBM are raised using veterinary progastrin-like molecule (VPM) from a veterinary source. Thus, the monoclonal antibodies can achieve the high affinity and specificity needed to diagnose, provide prognosis for, and treat progastrin-promoted diseases or conditions (GPD/C) in veterinary species. Applicant has also identified a group of VPM containing antigens for an immunogen. An immunogen containing the VPM are useful for the production of VPBM, including the production of VPBM against specific veterinary species, VPBM against multiple veterinary species, VPBM having low affinity to human progastrin, and combinations thereof.

### V. VPBM

Described herein are VPBM and systems containing VPBM specific for a veterinary species, VPBM and systems containing VPBM having binding affinity to a broad range of veterinary progastrin, VPBM having low affinity to human progastrin, and combinations thereof. In certain embodiments, the VPBM are developed using immunogens containing VPM specific to a veterinary species and/or VPM specific to multiple veterinary species. In certain embodiments, the VPM is a recombinant VPM. In certain embodiments, the VPBM are developed using immunogen containing VPM contain sequences that are not present human progastrin. In certain embodiments, the VPBM have broad specificity against VPM but has low binding affinity for human progastrin.

In certain embodiments, the VPBM can selectively bind to a VPM derived from one or more veterinary sources selected from the orders of Carnivora, Rodentia, Chiroptera, Artiodactyla, Perissodactyla, Cetacea, and Eulipotyphla.

In certain embodiments, the VPBM can selective bind to a VPM derived from one or more veterinary sources selected from Canis lupus familiaris, Vulpes vulpes, Vulpes lagopus, Phyllostomus discolor, Phyllostomus hastatus, Erinaceus europaeus, Felis catus, Panthera tigris, Lynx canadensis, Equus caballus, Equus asinus, Ailuropoda melanoleuca, Myotis davidii, Myotis brandtii, Myotis lucifugus, Eptesicus fuscus, Leopardus geoffroyi, Pipistrellus kuhlii, Panthera leo, Nyctereutes procyonoides, Lipotes vexillifer, Pteronotus parnellii mesoamericanus, Mus musculus, Mus caroli, Acomys russatus, Mesocricetus auratus, Microtus fortis, Cricetulus griseus, Mus pahari, Myodes glareolus, Microtus oregoni, Arvicola amphibius, Phodopus roborovskii, Onychomys torridus, Peromyscus maniculatus bairdii, Peromyscus californicus insignis, Mastomys coucha, Arvicanthis niloticus, Myotis myotis, Castor canadensis, Hyaena hyaena, Galemys pyrenaicus, Neophocaena asiaeorientalis asiaeorientalis, Delphinapterus leucas, Monodon monoceros, Phocoena sinus, Sousa chinensis, Lagenorhynchus obliquidens, Orcinus orca, Bos taurus, Ursus maritimus, Ursus americanus, Ovis ammon polii x Ovis aries, Cervus elaphus hippelaphus, Ovis aries, Budorcas taxicolor, Peromyscus leucopus, Marmota monax, Sciurus carolinensis, Marmota marmota marmota, Marmota flaviventris, Grammomys surdaster, Ictidomys tridecemlineatus, Miniopterus natalensis, Molossus molossus, Nannospalax galili, Rattus norvegicus, Rattus rattus, Ochotona princeps, Mirounga leonina, Halichoerus grypus, Leptonychotes weddellii, Mirounga angustirostris, Odobenus rosmarus divergens, Eumetopias jubatus, Callorhinus ursinus, Zalophus californianus, Lontra canadensis, Mustela erminea, Lemur catta, Manis javanica, Diceros bicornis minor, Ceratotherium simum simum, Canis lupus familiaris, Marmota monax, Nyctereutes procyonoides, Urocitellus parryii, Sus scrofa, Phacochoerus africanus, Phoca vitulina, Neogale vison, Mustela erminea, Lutra lutra, Enhydra lutris kenyoni, Meles meles, Mustela putorius furo, Tupaia chinensis, Propithecus coquereli, Microcebus murinus, Galeopterus variegatus, Manis pentadactyla, Manis javanica, Macaca nemestrina, Papio anubis, Cercocebus atys, Mandrillus leucophaeus, Macaca mulatta, Macaca fascicularis, Theropithecus gelada, Echinops telfairi, Otolemur gamettii, Orycteropus afer afer, Bubalus bubalis, Carlito syrichta, Pteronotus parnellii mesoamericanus, Vicugna pacos, Moschus berezovskii, Cervus canadensis, Odocoileus virginianus texanus, Bos taurus, Cervus elaphus, Oryx dammah, Muntiacus muntjak, Ovis aries, Muntiacus reevesi, Bos mutus, Capra hircus, Rangifer tarandus platyrhyncus, Cervus elaphus hippelaphus, Ovis ammon polii x Ovis aries, Gulo gulo, Felis catus, Lynx canadensis, Dasypus novemcinctus, Camelus ferus, Myotis myotis, Dipodomys spectabilis, Eschrichtius robustus, and combinations thereof.

In certain embodiments, the VPBM has preferred binding capacity to a VPM derived from one or more veterinary sources selected Table 12-Table 47, and combinations thereof. In certain embodiments, the VPBM has preferred binding capacity to a VPM derived from one or more veterinary sources selected from Table 12-Table 25, and combinations thereof. In certain embodiments, the VPBM has preferred binding capacity to a VPM derived from one or more veterinary sources selected from Table 26-Table 47, and combinations thereof. In certain preferred embodiments, the VPBM has preferred binding capacity to a VPM derived from one or more veterinary sources selected from Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain preferred embodiments, the VPBM has preferred binding capacity to a VPM derived from one or more veterinary sources selected from Table 12, Table 15, Table 16, Table 26, Table 30, Table 31, Table 32, Table 34, Table 40, and combinations thereof.

In certain preferred embodiments, the VPBM has preferred binding capacity to a VPM derived from one or more veterinary sources such as a canine (e.g., a dog), a feline (e.g., a cat, a tiger, and a lion), a bear (e.g., a panda and a polar bear), an equine (e.g., a horse and a donkey), a rodent (e.g., a mouse, a hamster, and a squirrel), a swine (e.g., a pig), a deer, a goat, a sheep, a cattle, and combinations thereof. In certain preferred embodiments, the VPBM has preferred binding capacity to a VPM derived from a non-human primate such as a macaque (e.g., a rhesus macaque and a lemur). In certain preferred embodiments, the VPBM has preferred binding capacity to a VPM derived from one or more domestic veterinary sources such as a domestic dog, a domestic cat, a domestic horse, a domestic donkey, a domestic pig, a domestic deer, a domestic goat, a domestic sheep, a domestic cattle, and combinations thereof. In certain embodiments, the VPBM has preferred binding capacity to a VPM derived from one or more laboratory non-human primate models such as a macaque (e.g. a rhesus macaque), a lemur, and combinations thereof. In certain embodiments, the VPBM has preferred binding capacity to a VPBM derived from one or more domestic companion veterinary sources, such as a dog, a cat, and a horse. In certain preferred embodiments, the VPBM has preferred binding capacity to a VPM derived from a domestic dog. In certain preferred embodiments, the VPBM has preferred binding capacity to a VPM derived from a domestic cat. In certain preferred embodiments, the VPBM has preferred binding capacity to a VPM derived from a domestic horse.

In certain embodiments, the VPBM has preferred binding capacity to a recombinant VPM. In certain embodiments, the VPBM has preferred binding capacity to a VPM having amino acid residues essentially the same as SEQ ID Nos 1, 2, 28-99, and combinations thereof. "Essentially the same as" as used herein means an amino acid residue that is the same as or has 1 amino acid that is different from the referenced sequences. In certain preferred embodiments, the VPBM has preferred binding capacity to a VPM having an amino acid residue essentially the same as SEQ ID Nos: 1, 30, 31, 32, 36, 37, 39, 52, 53, 55, 68, 72, 75, 81, 90, and combinations thereof. In certain embodiments, the VPBM has preferred binding capacity to a VPM having amino acid residues essentially the same as SEQ ID Nos: 78, 80, 83, 85 and combinations thereof. In certain preferred embodiments, the VPBM has preferred binding capacity to a VPM having amino acid residues essentially the same as SEQ ID Nos: 1, 30, 31, 68, and combinations thereof. In certain preferred embodiments, the VPBM has preferred binding capacity to a VPM having amino acid residues essentially the same as SEQ ID No: 1. In certain preferred embodiments, the VPBM has preferred binding capacity to a VPM having amino acid residues essentially the same as SEQ ID No: 68. In certain preferred embodiments, the VPBM has preferred binding capacity to a VPM having amino acid residues essentially the same as SEQ ID No: 30. In certain preferred embodiments, the VPBM has preferred binding capacity to a VPM having amino acid residue essentially the same as SEQ ID No: 31.

In certain embodiments, the VPBM binds to one or more amino acid residues on the sequence X₁SRLQDX₂PSGPGX₃, wherein X₁, X₂, X₃ is each a linker comprising between 0 to 40 residues, preferably between 0 to 35 residues, more preferably between 0 to 25 residues, more preferably between 0 to 20 residues, more preferably between 0 to 15 residues, more preferably between 0 to 14 residues, more preferably between 0 to 13 residues, more preferably between 0 to 12 residues, more preferably between 0 to 11 residues, more preferably between 0 to 10 residues, more preferably between 0 to 9 residues, more preferably between 0 to 8 residues, more preferably between 0 to 7 residues, more preferably between 0 to 6 residues, more preferably between 0 to 5 residues. In certain preferred embodiments, the linker X₁ X₂, and combinations thereof, does not interfere with the immunogenicity of the rest of the sequence. In certain embodiments, X₁ is SWKPR, X₂ is A, and/or X₃ is C or has no amino acid residue.

In certain embodiments, the VPBM binds to one or more amino acid residues on the sequence X₄WMEX₅EAAX₆AEEGDQRP, wherein X₄, X₅, and X₆ is each a linker comprising between 0 to 40 residues, preferably between 0 to 35 residues, more preferably between 0 to 25 residues, more preferably between 0 to 20 residues, more preferably between 0 to 15 residues, more preferably between 0 to 14 residues, more preferably between 0 to 13 residues, more preferably between 0 to 12 residues, more preferably between 0 to 11 residues, more preferably between 0 to 10 residues. In certain preferred embodiments, the linker X₄, X₅, and X₆, and combinations thereof, does not interfere with the immunogenicity of the rest of the sequence. In certain embodiments, X₄ is QGP or CQGP, X₅ is EE, and/or X₆ is YGWMDFGRRS.

In certain embodiments, a combination of VPBM form a system of VPBM to improve the binding specificity for VPM. In certain VPBM systems, a first VPBM binds to one or more amino acid residues on the sequence X₁SRLQDX₂PSGPGX₃; and a second VPBM binds to one or more amino acid residues on the sequence X₄WMEX₅EAAX₆AEEGDQRP. In certain embodiments, the linkers X₁, X₂, X₃, X₄, X₅, is a sequence comprising between 0 to 40 residues, preferably between 0 to 35 residues, more preferably between 0 to 25 residues, more preferably between 0 to 20 residues, more preferably between 0 to 15 residues, more preferably between 0 to 14 residues, more preferably between 0 to 13 residues, more preferably between 0 to 12 residues, more preferably between 0 to 11 residues, more preferably between 0 to 10 residues. In certain embodiments, each linker, whether alone or in combination with another linker, does not interfere with the immunogenicity of the rest of the first sequence and/or the second sequence. In certain embodiments, X₁ is SWKPR, X₂ is A, X₃ is C or has no amino acid residue, X₄ is QGP or CQGP, X₅ is EE, and/or X₆ is YGWMDFGRRS.

In certain embodiments, the VPBM binds to one or more veterinary-specific progastrin sequences (VPS), wherein the VPS include SRLQD, PSGPG, SRLQD, SGPG, RLQD, PG, LQD, PSGP, GPG, SPG, SGP, LQ, QD, WME, EAA, AEEGDQRP, EEGDQRP, DQRP, AEE, DQRP, AEEGDQR, AEEGDQ, AEEGD, AEEG, RP, GDQ, and GD. In certain embodiments, the VPBM binds to an epitope on a VPM on a region corresponding to (i) the N-terminal regions of human progastrin (amino acid residues 1-16), the region containing one or more veterinary specific N-terminus amino acid residue (N-VPS) and/or (ii) the C-terminal regions of human progastrin (amino acid residues 55-last amino acid), the region containing one or more veterinary specific C-terminus amino acid residue (C-VPS). A list of N-VPS includes SRLQD, PSGPG, SRLQD, SGPG, RLQD, PG, LQD, PSGP, GPG, SPG, SGP, LQ, and QD. A list of C-VPS includes WME, EAA, AEEGDQRP, EEGDQRP, DQRP, AEE, DQRP, AEEGDQR, AEEGDQ, AEEGD, AEEG, RP, GDQ, and GD. In certain preferred embodiments, the region contains both N-VPS and C-VPS. In certain preferred embodiments, the total number of amino acid residues from all VPS in the region is from 4 to 24, preferably from 5 to 24, preferably from 6 to 24, preferably from 7 to 24, preferably from 8 to 24, preferably from 9 to 24, preferably from 10 to 24, preferably from 11 to 24, preferably from 12 to 24, preferably from 13 to 24, preferably from 14 to 24, preferably from 15 to 24, preferably from 16 to 24, preferably from 17 to 24, preferably from 18 to 24, preferably from 19 to 24, preferably from 20 to 24, preferably from 21 to 24, preferably from 22 to 24, preferably from 23 to 24, and preferably 24.

In certain embodiments, the VPS is selected from the double underlined respective N-terminal or C-terminal amino acid residues in Table 12-Table 47, and combinations thereof. In certain embodiments, the VPS is selected the double underlined N-terminal amino acid residues in Table 12-Table 25, and combinations thereof. In certain embodiments, the VPS is selected from the double underlined C-terminal amino acid residues in Table 26-Table 47, and combinations thereof. In certain embodiments, the VPS is selected from the double underlined respective N-terminal or C-terminal amino acid residues in Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain embodiments, the VPS is selected from the double underlined respective N-terminal or C-terminal amino acid residues in Table 12, Table 15, Table 16, Table 26, Table 30, Table 31, Table 32, Table 34, Table 40. In certain preferred embodiments, the VPS is selected from the N-terminal amino acid residues SRLQD and PSGPG; LQD and PSGPG; and LQD and SGPG. In certain preferred embodiments, the VPS is selected from the C-terminal amino acid residues WME, EAA, and AEEGDQRP; WME and AEEGDQRP; EAA and AEEGDQRP; AEEGDQRP; WME and EAA; and EAA and AEEGDQ. In certain embodiments, the VPS contains the N-terminal amino acid residues SRLQD and PSGPG. In certain embodiments, the VPS contains the N-terminal amino acid residues LQD and PSGPG. In certain embodiments, the VPS contains the N-terminal amino acid residues LQD and SGPG. In certain preferred embodiments, the VPS contains the C-terminal amino acid residues WME, EAA, and AEEGDQRP. In certain preferred embodiments, the VPS contains the C-terminal amino acid residues WME and AEEGDQRP. In certain preferred embodiments, the VPS contains the C-terminal amino acid residues EAA and AEEGDQRP. In certain preferred embodiments, the VPS contains the C-terminal amino acid residues AEEGDQRP. In certain preferred embodiments, the VPS contains the C-terminal amino acid residues WME and EAA. In certain preferred embodiments, the VPS contains the C-terminal amino acid residues EAA and AEEGDQ. In certain preferred embodiments, the VPS contains the respective N-terminal and C-terminal amino acid residues SRLQD and PSGPG; and WME, EAA, and AEEGDQRP.

In certain embodiment the VPBM is a monoclonal antibody, preferably a purified monoclonal antibody. In certain embodiment the VPBM is a binding domain of a monoclonal antibody, such as a heavy chain and/or a light chain. The binding domain is preferably purified. In certain embodiments, the monoclonal antibody and/or the binding domain of the monoclonal antibody is encoded by a DNA sequence listed in Table 4-Table 11. In certain embodiments, the monoclonal antibody and/or the binding domain is a heavy chain encoded by a DNA sequence listed in Table 4, Table 6, Table 8, and Table 10. In certain embodiments, the monoclonal antibody and/or the binding domain is a light chain encoded by a DNA sequence listed in Table 5, Table 7, Table 9, and Table 11. In certain embodiments, the monoclonal antibody and/or the binding domain binds to the N terminus of the VPM is encoded by a DNA sequence listed in Table 4, Table 5, Table 8, and Table 9. In certain embodiments, the monoclonal antibody and/or the binding domain binds to the C terminus of the VPM is encoded by a DNA sequence listed in Table 6, Table 7, Table 10, and Table 11.

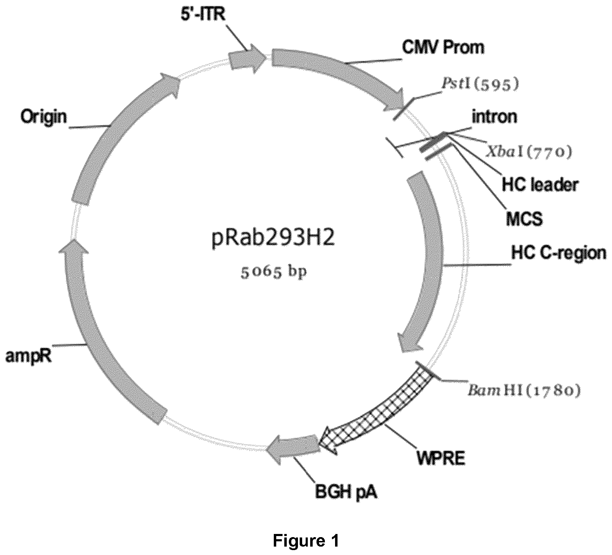

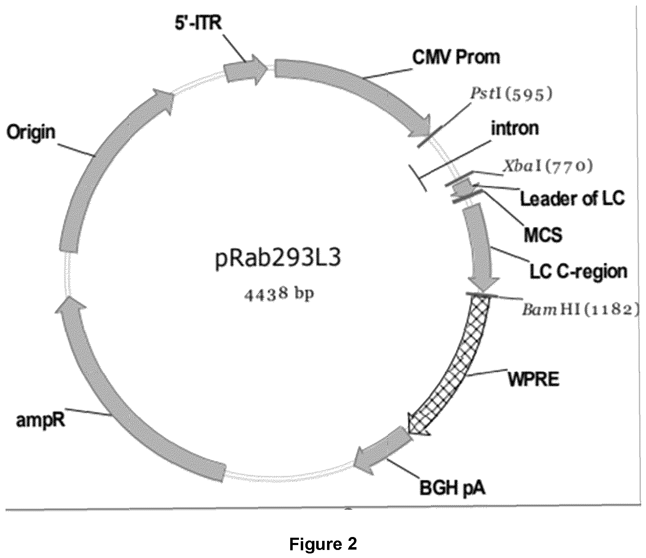

In certain embodiments, the VPBM is encoded by a vector plasmid containing a sequence listed in Table 8-Table 11. In certain embodiments, vector plasmid is suitable for making the VPBM described herein. In certain embodiments, the vector comprise the nucleic acids sequences of SEQ ID NO: 12-27, and combinations thereof. In certain embodiments, the nucleic acids are encoded in a pRab293 vector and transfected into Expi293F cells, to produce the VPBM. In other embodiments a prokaryotic and/or eukaryotic cloning vector and corresponding cell can be used to produce the VPBM. Schematic examples of the vector can be found in Figure 1 and Figure 2.

Also described herein are hybridomas for making the VPBM. Hybridomas A hybridoma is an immortal hybrid cell line, produced by fusing a B lymphocyte, such as a spleen cell, with cancerous B cell, such as a myeloma cell. The B lymphocyte contains the nucleic acid residues encoding the VPBM and when fused with the cancerous B cell, the resulting hybridoma can be used to produce the VPBM. Hybridomas with the desirable properties as described herein can be clonally selected and expanded in tissue culture for the production of the VPBM. Supernatants of the hybridomas can be purified to obtain the VPBM. The VPBM can be used directly as needed or combined with a suitable diluent. The VPBM can be combined with one or more different VPBM and/or mixed with a buffer, excipient and/or preservative suitable for veterinary diagnosis, prognosis, and/or treatment.

### VI. Recombinant and Chimeric VPBM

In certain embodiments, VPBM is a recombinant VPBM. the In certain embodiments, the recombinant VPBM is a chimeric monoclonal antibody having a Fc region selected from the same veterinary species as a subject being treated, diagnosed, or tested using the VPBM. For example, a VPBM intended for use in dogs can have a Fc region of a dog. As another example, a VPBM intended for use in cats can have a Fc region of a cat. As another example, a VPBM intended for use in horses can have a Fc region of a horse. In certain embodiments, the Fc region will elicit low non-specific host response when administered to the veterinary subject. In certain embodiments, the non-specific host response elicited by the Fc region in a veterinary subject is less than 50% of that of a human Fc region, preferably less than 40%, more preferably less than 30%, more preferably less than 20%, more preferably less than 10%, more preferably less than 5%, more preferably less than 1%, more preferably less than 0.1%, more preferably less than 0.01%. In certain preferred embodiment, the VPBM has no Fc region.

In certain embodiments, the VPBM is a bivalent chimeric VPBM, that is, a chimeric VPBM having two different sites for VPM. Exemplary bivalent chimeric VPBM includes a first binding site for the N terminus of the VPM and a second binding site for the C terminus of the VPM. In certain embodiment, the first binding site of the VPBM binds to the VPS at the N-terminus, including SRLQD, PSGPG, SRLQD, SGPG, RLQD, PG, LQD, PSGP, GPG, SPG, SGP, LQ, QD, and combinations thereof; and the second binding site of the VPBM binds to VPS at the C-terminus, including WME, EAA, AEEGDQRP, EEGDQRP, DQRP, AEE, DQRP, AEEGDQR, AEEGDQ, AEEGD, AEEG, RP, GDQ, GD, and combinations thereof. In certain preferred embodiments, the total number of amino acid residues from all VPS from both binding sites is from 4 to 24, preferably from 5 to 24, preferably from 6 to 24, preferably from 7 to 24, preferably from 8 to 24, preferably from 9 to 24, preferably from 10 to 24, preferably from 11 to 24, preferably from 12 to 24, preferably from 13 to 24, preferably from 14 to 24, preferably from 15 to 24, preferably from 16 to 24, preferably from 17 to 24, preferably from 18 to 24, preferably from 19 to 24, preferably from 20 to 24, preferably from 21 to 24, preferably from 22 to 24, preferably from 23 to 24, and preferably 24.

In certain embodiments, the first binding site for the N terminus of the bivalent chimeric VPM is selected the double underlined N-terminal amino acid residues in Table 12-Table 25, and combinations thereof. In certain embodiments, the second binding site for the C terminus of the VPM is selected from the double underlined C-terminal amino acid residues in Table 26-Table 47, and combinations thereof. In certain embodiments, the VPS for the first and second binding site is selected from the double underlined respective N-terminal or C-terminal amino acid residues in Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain embodiments, the VPS for the first and second binding site is selected from the double underlined respective N-terminal or C-terminal amino acid residues in Table 12, Table 15, Table 16, Table 26, Table 30, Table 31, Table 32, Table 34, Table 40. In certain preferred embodiments, the VPS for the first binding site is selected from the N-terminal amino acid residues SRLQD and PSGPG; LQD and PSGPG; and LQD and SGPG. In certain preferred embodiments, the VPS for the second binding site is selected from the C-terminal amino acid residues WME, EAA, and AEEGDQRP; WME and AEEGDQRP; EAA and AEEGDQRP; AEEGDQRP; WME and EAA; and EAA and AEEGDQ. In certain embodiments, the VPS for the first binding site contains the N-terminal amino acid residues SRLQD and PSGPG. In certain embodiments, the VPS for the first binding site contains the N-terminal amino acid residues LQD and PSGPG. In certain embodiments, the VPS for the first binding site contains the N-terminal amino acid residues LQD and SGPG. In certain preferred embodiments, the VPS for the second binding site contains the C-terminal amino acid residues WME, EAA, and AEEGDQRP. In certain preferred embodiments, the VPS for the second binding site contains the C-terminal amino acid residues WME and AEEGDQRP. In certain preferred embodiments, the VPS for the second binding site contains the C-terminal amino acid residues EAA and AEEGDQRP; AEEGDQRP. In certain preferred embodiments, the VPS for the second binding site contains the C-terminal amino acid residues WME and EAA. In certain preferred embodiments, the VPS for the second binding site contains the C-terminal amino acid residues EAA and AEEGDQ. In certain preferred embodiments, the VPS for the first and second binding site contains the respective N-terminal and C-terminal amino acid residues SRLQD and PSGPG; and WME, EAA, and AEEGDQRP.

In certain embodiments, an effective dose of the chimeric VPBM can treat a GPD/C. In certain embodiments, the bivalent chimeric VPBM is superior to a monovalent VPBM in treating a GPD/C at a lower dose. In certain embodiments, the bivalent chimeric VPBM is superior to a monovalent VPBM in treating a GPD/C with lower toxicity and/or adverse effects. In certain embodiments, the bivalent chimeric VPBM is more effective at treating a GPD/C than a monovalent VPBM. In certain embodiments, pursuant to the disclosures herein, a bivalent chimeric VPBM elicits a lower non-specific host response following administer to veterinary subject when compared to a human based progastrin binding molecule.

In certain embodiment the bivalent chimeric VPBM a purified chimeric antibody. In certain embodiments, the first and second binding domain of the chimeric antibody is encoded by a DNA sequence listed in Table 4-Table 11. In certain embodiments, the first binding domain is a heavy chain encoded by a DNA sequence listed in Table 4, Table 6, Table 8, and Table 10. In certain embodiments, the second binding domain is a light chain encoded by a DNA sequence listed in Table 5, Table 7, Table 9, and Table 11. In certain embodiments, the first binding domain binds to the N terminus of the VPM is encoded by a DNA sequence listed in Table 4, Table 5, Table 8, and Table 9. In certain embodiments, the second binding domain binds to the C terminus of the VPM is encoded by a DNA sequence listed in Table 6, Table 7, Table 10, and Table 11.

In certain embodiments, the chimeric VPBM is capable of binding different VPM. In certain preferred embodiments, the VPBM is capable of accurately and selectively bind to one VPBM; preferably, different VPM from two veterinary species; more preferably different VPM from three veterinary species; more preferably, different VPM from four veterinary species; more preferably, different VPM from five veterinary species; more preferably, different VPM from six veterinary species; more preferably, different VPM from seven veterinary species; more preferably, different VPM from eight veterinary species; more preferably, different VPM from nine veterinary species; more preferably, different VPM from ten veterinary species; more preferably, different VPM from ten veterinary species; more preferably, different VPM from 11 veterinary species; more preferably, different VPM from 12 veterinary species; more preferably, different VPM from 13 veterinary species; more preferably, different VPM from 14 veterinary species; more preferably, different VPM from 15 veterinary species; more preferably, different VPM from 16 veterinary species; more preferably, different VPM from 17 veterinary species; more preferably, different VPM from 18 veterinary species; more preferably, different VPM from 19 veterinary species; more preferably, different VPM from 20 veterinary species; more preferably, different VPM from 21 veterinary species; more preferably, different VPM from 22 veterinary species; more preferably, different VPM from 23 veterinary species; more preferably, different VPM from 24 veterinary species; more preferably, different VPM from 25 veterinary species; more preferably, different VPM from 26 veterinary species; more preferably, different VPM from 27 veterinary species; more preferably, different VPM from 28 veterinary species; more preferably, different VPM from 29 veterinary species; more preferably, different VPM from 30 veterinary species; more preferably, different VPM from 31 veterinary species; more preferably, different VPM from 32 veterinary species; more preferably, different VPM from 33 veterinary species; more preferably, different VPM from 34 veterinary species; more preferably, different VPM from 35 veterinary species; more preferably, different VPM from 36 veterinary species; more preferably, different VPM from 37 veterinary species; more preferably, different VPM from 38 veterinary species; more preferably, different VPM from 39 veterinary species; more preferably, different VPM from 40 veterinary species; more preferably, different VPM from 41 veterinary species; more preferably, different VPM from 42 veterinary species; more preferably, different VPM from 43 veterinary species; more preferably, different VPM from 44 veterinary species; more preferably, different VPM from 45 veterinary species; more preferably, different VPM from 46 veterinary species; more preferably, different VPM from 47 veterinary species; more preferably, different VPM from 48 veterinary species; more preferably, different VPM from 49 veterinary species; more preferably, different VPM from 50 veterinary species; more preferably, different VPM from about 50 to about 75 species; more preferably, different VPM from about 75 to about 100 species; more preferably, different VPM from about 100 to about 125 species; more preferably, different VPM from about 100 to about 138 species;. and more preferably, different VPM from about 125 to about 150 species. In certain embodiments, the VPBM is capable of binding to different VPM from about 2 to about 500 different species. In certain embodiments, a non-chimeric monoclonal antibody and/or a binding domain of a monoclonal antibody is also capable of accurately and selectively binding to different VPBMs as described in this paragraph. In certain embodiments, a composition comprising a mixture of a plurality of chimeric VPBM, monoclonal antibodies, and binding domains of VPM, wherein the mixture is also capable binding to different VPM as described in this paragraph. In certain embodiments, the chimeric VPBM, monoclonal antibodies, binding domains against VPM, and mixtures thereof, is also capable of diagnosing, monitoring, and treating one or more GPD/C from a the range of different veterinary species described in this paragraph.

In certain embodiments, the chimeric VPBM is encoded by a vector plasmid containing one or more sequence listed in Table 8-Table 11. In certain embodiments, vector plasmid is suitable for making the chimeric VPBM described herein. In certain embodiments, the vector comprise the nucleic acids sequences of SEQ ID NO: 12-27, and combinations thereof. In certain embodiments, the nucleic acids are encoded in a pRab293 vector and transfected into Expi293F cells, to produce the VPBM. In other embodiments, a prokaryotic and/or eukaryotic cloning vector and corresponding cell can be used to produce the VPBM.

### VII. A VPBM system

Also disclosed herein is a VPBM system comprised of one or more VPBM. In certain embodiments, the VPBM system is suitable for providing diagnosis and/or prognosis for a GPD/C. In certain embodiments, the VPBM system comprise a first VPBM containing a binding site for the N terminus of the VPM and a second VPBM that contains a binding site for the C terminus of the VPM. In certain embodiment, the binding site of the first VPBM binds to the VPS at the N-terminus, including SRLQD, PSGPG, SRLQD, SGPG, RLQD, PG, LQD, PSGP, GPG, SPG, SGP, LQ, QD, and combinations thereof; and the binding site of the second VPBM binds to VPS at the C-terminus, including WME, EAA, AEEGDQRP, EEGDQRP, DQRP, AEE, DQRP, AEEGDQR, AEEGDQ, AEEGD, AEEG, RP, GDQ, GD, and combinations thereof. In certain preferred embodiments, the total number of amino acid residues from all VPS from both binding sites is from 4 to 24, preferably from 5 to 24, preferably from 6 to 24, preferably from 7 to 24, preferably from 8 to 24, preferably from 9 to 24, preferably from 10 to 24, preferably from 11 to 24, preferably from 12 to 24, preferably from 13 to 24, preferably from 14 to 24, preferably from 15 to 24, preferably from 16 to 24, preferably from 17 to 24, preferably from 18 to 24, preferably from 19 to 24, preferably from 20 to 24, preferably from 21 to 24, preferably from 22 to 24, preferably from 23 to 24, and preferably 24.

In certain embodiments, the binding site of the first VPBM binds is selected from the double underlined N-terminal amino acid residues in Table 12-Table 25, and combinations thereof. In certain embodiments, the binding site of the second VPBM is selected from the double underlined C-terminal amino acid residues in Table 26-Table 47, and combinations thereof. In certain embodiments, the VPS for the first VPBM and second VPBM is selected from the double underlined respective N-terminal or C-terminal amino acid residues in Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain embodiments, the VPS for the first and second VPBM is selected from the double underlined respective N-terminal or C-terminal amino acid residues in Table 12, Table 15, Table 16, Table 26, Table 30, Table 31, Table 32, Table 34, Table 40. In certain preferred embodiments the VPS for the first VPBM is selected from the N-terminal amino acid residues SRLQD and PSGPG; LQD and PSGPG; and LQD and SGPG. In certain preferred embodiments, the VPS for the second VPBM is selected from the C-terminal amino acid residues WME, EAA, and AEEGDQRP; WME and AEEGDQRP; EAA and AEEGDQRP; AEEGDQRP; WME and EAA; and EAA and AEEGDQ. In certain embodiments, the VPS for the first VPBM contains the N-terminal amino acid residues SRLQD and PSGPG. In certain embodiments, the VPS for the first VPBM contains the N-terminal amino acid residues LQD and PSGPG. In certain embodiments, the VPS for the first VPBM contains the N-terminal amino acid residues LQD and SGPG. In certain preferred embodiments, the VPS for the second VPBM contains the C-terminal amino acid residues WME, EAA, and AEEGDQRP. In certain preferred embodiments, the VPS for the second VPBM contains the C-terminal amino acid residues WME and AEEGDQRP. In certain preferred embodiments, the VPS for the second VPBM contains the C-terminal amino acid residues EAA and AEEGDQRP; AEEGDQRP. In certain preferred embodiments, the VPS for the second VPBM contains the C-terminal amino acid residues WME and EAA. In certain preferred embodiments, the VPS for the second VPBM contains the C-terminal amino acid residues EAA and AEEGDQ. In certain preferred embodiments, the VPS for the first and second VPBM contains the respective N-terminal and C-terminal amino acid residues SRLQD and PSGPG; and WME, EAA, and AEEGDQRP.

In certain embodiments, the VPBM system is used in a sandwich enzyme-linked immunosorbent assay (**ELISA**). In certain embodiments, a coating VPBM is first absorbed onto the surface of a well in an ELISA plate; a sample, such as a biological sample, which may contain a VPM, is then added to the same well in the ELISA plate; then a detection VPBM is added to detect whether a VPM is present in the sample. In certain embodiments, the detection VPBM may be conjugated to a detection agent, such as a horseradish peroxidase (**HRP**), biotin, alkaline phosphatase (**AP**), β-galactosidase, acetylcholinesterase, luciferase, and urease, among others. The well in the ELISA plate is then measured for VPBM is visualized using a second detection agent, if needed. The second detection agent can include TMB (3,3',5,5'-Tetramethylbenzidine), OPD (o-phenylenediamine dihydrochloride), ABTS (2,2'-Azino-bis(3-ethylbenzothiazoline-6-sulphonic acid)), Amplex Red, and Luminol, when the first detection agent is HRP; avidin and streptavidin, when the first detection agent is biotin; p-Nitrophenyl phosphate (pNPP), 5-Bromo-4-chloro-3-indolyl phosphate/Nitro blue tetrazolium (BCIP/NBT), 4-Methylumbelliferyl phosphate (4-MUP), CSPD, and CDP-Star when the first detection agent is AP; o-Nitrophenyl β-D-galactopyranoside (ONPG) and 4-Methylumbelliferyl β-D-galactopyranoside (MUG), when the first detection agent is β-galactosidase; acetylthiocholine, when the first detection agent is acetylcholinesterase; luciferin, when the first detection agent is luciferase; and urea, when the first detection agent is urease. In certain embodiments, the coating VPBM is the first VPBM in the system and the detection VPBM is the second VPBM in the system. In certain embodiments, the coating VPBM is the second VPBM in the system and the detection VPBM is the first VPBM in the system. In certain embodiment, the detection VPBM is not conjugated to a detection system and a secondary detection molecule specific for the detection VPBM is added. The secondary detection molecule can be conjugated to the first detection agent and produce a signal in the presence of the second detection agent as described herein.

In certain embodiments, the VPBM system is used in a multiplex ELISA. In certain embodiments the coating VPBM is a combination of one or more first VPBM and one or more second VPBM; and the detection VPBM is a combination of one or more first VPBM and one or more second VPBM. The coating VPBM and the detection VPBM can be the same.

In certain embodiments, the coating VPBM can be bound to a bead, such as a glass or magnetic bead, to detect a sample, such as a biological sample, which may contain a VPM. In certain embodiments, such as a western blot, the coating VPBM is used to precipitate any VPM from a sample, and the detection VPBM is used to detect the presence of any VPM. In certain embodiments, the VPBM system is able to detect a concentration of VPM at or above the threshold level for a GPD/C.

In certain embodiments, an effective dose of the VPBM system can treat a GPD/C. In certain embodiments, the VPBM system is superior to a VPBM in treating a GPD/C at a lower dose. In certain embodiments, the VPBM system is superior to a VPBM in treating a GPD/C with lower toxicity and/or adverse effects. In certain embodiments, the VPBM system is more effective at treating a GPD/C than a VPBM. In certain embodiments, pursuant to the disclosures herein, the VPBM system elicits a lower non-specific host response following administer to veterinary subject when compared to one or more human based progastrin binding molecule.

### VIII. VPBM as method of veterinary treatments

Neutralizing VPBM can bind and modulate and/or inhibit veterinary progastrin and/or gastrin functions and/or signaling. In certain embodiments, neutralizing VPBM can yield a statistically significant reduction in veterinary tumor progression and veterinary cancer progression as compared to a non-specific monoclonal antibody. In certain embodiments, the neutralizing VPBM exhibits a statistically significant veterinary therapeutic benefit (i.e., tumor reduction, cancer cell reduction, or other veterinary health profiles) of at least about 10%, preferably at least about 15%, preferably at least about 20%, preferably at least about 30%, preferably at least about 40%, and preferably at least about 50%.

In certain embodiments, the VPBM is in a pharmaceutical composition comprising one or more pharmacologically acceptable non-toxic carriers, excipients, diluent, and/or preservative.

In certain embodiments, the VPBM has a therapeutic veterinary affinity of at least about 100 nM. In certain embodiments, the VPBM has a therapeutic veterinary affinity selected from the range: from about 90 nM about 0.001 nM, from about 80 nM to about 0.001 nM, from about 70 nM to about 0.001 nM, from about 60 nM to about 0.001 nM, from about 50 nM to about 0.001 nM, from about 40 nM to about 0.001 nM, from about 30 nM to about 0.001 nM, from about 25 nM to about 0.001 nM, from about 20 nM to about 0.001 nM, from about 15 nM to about 0.001 nM, from about 10 nM to about 0.001 nM, from about 7 nM to about 0.001 nM, from about 6 nM to about 0.001 nM, from about 5 nM to about 0.001 nM, from about 4 nM to about 0.001 nM, from about 3 nM to about 0.001 nM, from about 2 nM to about 0.001 nM, from about 1 nM to about 0.001 nM, from about 0.2 nM to about 0.001 nM, from about 0.1 nM to about 0.001 nM, from about 0.09 nM to about 0.001 nM, from about 0.08 nM to about 0.001 nM, from about 0.07 nM to about 0.001 nM, from about 0.06 nM to about 0.001 nM, from about 0.05 nM to about 0.001 nM, from about 0.04 nM to about 0.001 nM, from about 0.03 nM to about 0.001 nM, from about 0.02 nM to about 0.001 nM, from about 0.01 nM to about 0.001 nM, from about 0.009 nM to about 0.001 nM, from about 0.008 nM to about 0.001 nM, from about 0.007 nM to about 0.001 nM, from about 0.006 nM to about 0.001 nM, from about 0.005 nM to about 0.001 nM, from about 0.004 nM to about 0.001 nM, from about 0.003 nM to about 0.001 nM, and from about 0.002 nM to about 0.001 nM. In certain embodiments, the VPBM has a value of therapeutic veterinary affinity below 0.001 nM.

In certain embodiments, the VPBM has a therapeutic veterinary affinity of at least about 1600 ng/mL. In certain embodiments, the VPBM has a therapeutic veterinary affinity selected from the range: from about 1400 ng/mL to about 0.1 ng/mL, about 1200 ng/mL to about 0.1 ng/mL, from about 1000 ng/mL to about 0.1 ng/mL, from about 800 ng/mL to about 0.1 ng/mL, from about 600 ng/mL to about 0.1 ng/mL, from about 400 ng/mL to about 0.1 ng/mL, from about 200 ng/mL to about 0.1 ng/mL, from about 180 ng/mL to about 0.1 ng/mL, from about 160 ng/mL to about 0.1 ng/mL, from about 140 ng/mL to about 0.1 ng/mL, from about 120 ng/mL to about 0.1 ng/mL, from about 100 ng/mL to about 0.1 ng/mL, from about 80 ng/mL to about 0.1 ng/mL, from about 60 ng/mL to about 0.1 ng/mL, from about 40 ng/mL to about 0.1 ng/mL, from about 20 ng/mL to about 0.1 ng/mL, from about 16 ng/mL to about 0.1 ng/mL, from about 10 ng/mL to about 0.1 ng/mL, from about 8 ng/mL to about 0.1 ng/mL, from about 6 ng/mL to about 0.1 ng/mL, from about 4 ng/mL to about 0.1 ng/mL, from about 2 ng/mL to about 0.1 ng/mL, from about 1.8 ng/mL to about 0.1 ng/mL, from about 1.6 ng/mL to about 0.1 ng/mL, from about 1.4 ng/mL to about 0.1 ng/mL, from about 1.2 ng/mL to about 0.1 ng/mL, from about 1.0 ng/mL to about 0.1 ng/mL, from about 0.8 ng/mL to about 0.1 ng/mL, from about 0.6 ng/mL to about 0.1 ng/mL, from about 0.4 ng/mL to about 0.1 ng/mL, from about 0.2 ng/mL to about 0.1 ng/mL, and from about 0.16 ng/mL to about 0.1 ng/mL. In certain embodiments the VPBM has a value of therapeutic veterinary affinity value below about 0.1 ng/mL.

In certain embodiments, the VPBM is capable of treating a GPD/C in a veterinary subject having a progastrin level at least about 2.5 times the normal progastrin level. Preferably the progastrin level of the veterinary subject is selected from the range: from about 5 times to about 500 times of the normal level; from about 7.5 times to about 500 times of the normal level; from about 10 times to about 500 times of the normal level; from about 12.5 times to about 500 times of the normal level; from about 15 times to about 500 times of the normal level; from about 17.5 times to about 500 times of the normal level; from about 20 times to about 500 times of the normal level; from about 20 times to about 500 times of the normal level; from about 25 times to about 500 times of the normal level; from about 30 times to about 500 times of the normal level; from about 35 times to about 500 times of the normal level; from about 40 times to about 500 times of the normal level; from about 45 times to about 500 times of the normal level; from about 50 times to about 500 times of the normal level; from about 55 times to about 500 times of the normal level; from about 60 times to about 500 times of the normal level; from about 65 times to about 500 times of the normal level; from about 70 times to about 500 times of the normal level; from about 75 times to about 500 times of the normal level; from about 80 times to about 500 times of the normal level; from about 85 times to about 500 times of the normal level; from about 90 times to about 500 times of the normal level; from about 95 times to about 500 times of the normal level; from about 100 times to about 500 times of the normal level; from about 125 times to about 500 times of the normal level; from about 150 times to about 500 times of the normal level; from about 175 times to about 500 times of the normal level; from about 200 times to about 500 times of the normal level; from about 250 times to about 500 times of the normal level; from about 300 times to about 500 times of the normal level; from about 350 times to about 500 times of the normal level; from about 400 times to about 500 times of the normal level; and from about 450 times to about 500 times of the normal level. In certain embodiments, the VPBM is capable of treating a GPD/C in a veterinary subject having a progastrin level exceeding 500 times the normal progastrin level.

In certain embodiments, the affinity of VPBM can be determined by ELISA, isothermal titration calorimetry (ITC), and/or fluorescent polarization assay. In certain embodiments, the VPBM has a strong affinity to a dprogastrin-like protein (such as a dprogastrin-SUMO protein) and the VPBM has a low affinity to a human progastrin. In certain embodiments, the VPBM has a strong affinity to the protein having the sequence of SEQ ID NO: 1 and/or SEQ ID NO: 2 and has a low affinity to the protein having the sequence of SEQ ID NO: 5 and/or SEQ ID NO: 6.

In certain embodiments, the VPBM has an affinity constant to a dprogastrin-like protein (such as a dprogastrin-SUMO protein), the affinity constant selected from the range: from about 1×10⁻⁶ M to about 1×10⁻¹⁶ M, from about 5×10⁻⁷ M to about 1×10⁻¹⁶ M, from about 1×10⁻⁷M to about 1×10⁻¹⁶M, from about 5×10⁻⁸ M to about 1×10⁻¹⁶M, from about 1×10⁻⁸ M to about 1×10⁻¹⁶M, from about 5×10⁻⁹ M to about 1×10⁻¹⁶M, from about 1×10⁻⁹ M to about 1×10⁻¹⁶M, from about 5×10⁻¹⁰ M to about 1×10⁻¹⁶M, from about 1×10⁻¹⁰ M to about 1×10⁻¹⁶M, from about 5×10⁻¹¹ M to about 1×10⁻¹⁶M, from about 1×10⁻¹¹ M to about 1×10⁻¹⁶ M, from about 5×10⁻¹² M to about 1×10⁻¹⁶ M, and from about 1×10⁻¹² to about 1×10⁻¹⁶ M.

The pharmaceutical composition can be formulated for administration parenterally, subcutaneously, and intravenously, among others. In certain embodiment the VPBM or the pharmaceutical composition comprising VPBM is used to treat a GPD/C. A veterinary subject diagnosed with a GPD/C can be administered a therapeutic effective amount of VPBM. A veterinary subject can be a mammal selected from the orders of Carnivora, Rodentia, Chiroptera, Artiodactyla, Perissodactyla, Cetacea, and Eulipotyphla. In certain embodiments, the veterinary subject is selected from Table 12-Table 47. In certain embodiments, the veterinary subject is selected from Table 12-Table 25, and combinations thereof. In certain embodiments, the veterinary subject is selected from Table 26-Table 47, and combinations thereof. In certain embodiments, the veterinary subject is selected from Table 12, Table 13, Table 14, Table 15, Table 16, Table 26, Table 27, Table 28, Table 30, Table 31, Table 32, Table 34, Table 35, Table 36, Table 37, Table 38, Table 40, and combinations thereof. In certain embodiments, the veterinary subject is selected from Table 12, Table 15, Table 16, Table 26, Table 30, Table 31, Table 32, Table 34, Table 40, and combinations thereof.

In certain embodiments, the veterinary subject is selected from a mammal such as a canine (e.g., a dog), a feline (e.g., a cat, a tiger, and a lion), a bear (e.g. a panda and a polar bear), an equine (e.g. a horse and a donkey), a rodent (e.g., a mouse, a hamster, and a squirrel), a swine (e.g., a pig), and a deer, a goat, a sheep, a cattle, and combinations thereof. In certain embodiments, the veterinary subject is selected from a non-human primate such as a macaque (e.g., a rhesus macaque and a lemur). In certain embodiments, the veterinary subject is selected from domestic veterinary sources such as a domestic dog, a domestic cat, a domestic horse, a domestic donkey, a domestic pig, a domestic deer, a domestic goat, a domestic sheep, a domestic cattle, and combinations thereof. In certain embodiments, the veterinary subject is selected from one or more laboratory non-human primate models such as a macaque (e.g. a rhesus macaque), a lemur, and combinations thereof. In certain embodiments, the veterinary subject is a domestic dog. In certain embodiments, the veterinary subject is a domestic cat. In certain embodiments, the veterinary subject is a domestic horse.

In certain embodiments, two or more VPBM is used to treat the GPD/C. In certain embodiments, three or more VPBM are used to treat the GPD/C. In certain embodiments, three or more VPBM are used to treat the GPD/C. In certain embodiments, four or more VPBM are used to treat the GPD/C. In certain embodiments, a combination of a VPBM VPM N-terminus and a VPBM specific for the C-terminus are used to treat the GPD/C. In certain embodiments, two pairs of a VPBM VPM N-terminus and a VPBM specific for the C-terminus are used to treat the GPD/C. In certain embodiments, the combination of VPBM achieves the therapeutic benefits for a GPD/V in a veterinary subject described herein.

In certain embodiments, a chimeric VPBM as described herein is used to treat the GPD/C. In certain embodiments, chimeric VPBM achieves the binding affinity and/or the affinity constant described herein. In certain embodiments, the chimeric VPBM achieves the therapeutic benefits for a GPD/V in a veterinary subject described herein.

The veterinary treatment described here can be combined with, or prescribed in conjunction with one or more additional therapy, such as chemotherapeutic treatment, radiation therapy, surgery, and antibody therapy, among others.

### IX. VPBM as methods of veterinary diagnostics

The VPBM described herein can bind to circulating VPM in the bodily fluids of a veterinary subject and can be used in a method to diagnose, provide prognosis, and/or monitor the progression of a GPD/C. In certain embodiments, the VPBM can accurately detect low amounts of circulating VPM in a veterinary subject with statistical significance. A diagnostic kit comprising a VPBM and an instruction manual can be used to provide diagnosis and/or prognosis of a GPD/C. In certain embodiments, the diagnostic kit is used to detect a GPD/C from a veterinary subject. Suitable veterinary subjects are previously described. In certain embodiment, a method of diagnosing and/or prognosing a GPD/C in a veterinary subject includes using a VPBM to detect the presence or absence of a VPM in a veterinary subject in need thereof and determining whether the amount of VPM is above or below the threshold of the of GPD/C, wherein an amount of VPM above the threshold of GPD/C is indicative of a GPD/C.

In certain embodiments, a monitoring kit comprising a VPBM and an instruction manual is used to monitor the progression of GPD/C in a veterinary subject. In certain embodiments, In certain embodiments, level of VPM in a sample from a veterinary subject is determined using a VPBM and the level of VPM at different time points are compared. An increase in VPM levels is indicative of a worsening of the GPD/C and a reduction in VPM levels is indicative of a recovery of the GPD/C.

In certain embodiments, the VPM is a circulating VPM derived from the bodily fluids of a veterinary subject. In certain embodiments, the bodily fluids is a blood sample, a serum sample, a mucosal fluid sample, and/or an urine sample. In certain embodiments, the threshold level of the VPM is selected from the range: from about 90 nM about 0.001 nM, from about 80 nM to about 0.001 nM, from about 70 nM to about 0.001 nM, from about 60 nM to about 0.001 nM, from about 50 nM to about 0.001 nM, from about 40 nM to about 0.001 nM, from about 30 nM to about 0.001 nM, from about 25 nM to about 0.001 nM, from about 20 nM to about 0.001 nM, from about 15 nM to about 0.001 nM, from about 10 nM to about 0.001 nM, from about 7 nM to about 0.001 nM, from about 6 nM to about 0.001 nM, from about 5 nM to about 0.001 nM, from about 4 nM to about 0.001 nM, from about 3 nM to about 0.001 nM, from about 2 nM to about 0.001 nM, from about 1 nM to about 0.001 nM, from about 0.2 nM to about 0.001 nM, from about 0.1 nM to about 0.001 nM, from about 0.09 nM to about 0.001 nM, from about 0.08 nM to about 0.001 nM, from about 0.07 nM to about 0.001 nM, from about 0.06 nM to about 0.001 nM, from about 0.05 nM to about 0.001 nM, from about 0.04 nM to about 0.001 nM, from about 0.03 nM to about 0.001 nM, from about 0.02 nM to about 0.001 nM, from about 0.01 nM to about 0.001 nM, from about 0.009 nM to about 0.001 nM, from about 0.008 nM to about 0.001 nM, from about 0.007 nM to about 0.001 nM, from about 0.006 nM to about 0.001 nM, from about 0.005 nM to about 0.001 nM, from about 0.004 nM to about 0.001 nM, from about 0.003 nM to about 0.001 nM, and from about 0.002 nM to about 0.001 nM..

In certain embodiments, the VPBM has a diagnostic veterinary affinity of at least about 1600 ng/mL. In certain embodiments, the VPBM has a diagnostic veterinary affinity selected from the range: from about 1400 ng/mL to about 0.1 ng/mL, about 1200 ng/mL to about 0.1 ng/mL, from about 1000 ng/mL to about 0.1 ng/mL, from about 800 ng/mL to about 0.1 ng/mL, from about 600 ng/mL to about 0.1 ng/mL, from about 400 ng/mL to about 0.1 ng/mL, from about 200 ng/mL to about 0.1 ng/mL, from about 180 ng/mL to about 0.1 ng/mL, from about 160 ng/mL to about 0.1 ng/mL, from about 140 ng/mL to about 0.1 ng/mL, from about 120 ng/mL to about 0.1 ng/mL, from about 100 ng/mL to about 0.1 ng/mL, from about 80 ng/mL to about 0.1 ng/mL, from about 60 ng/mL to about 0.1 ng/mL, from about 40 ng/mL to about 0.1 ng/mL, from about 20 ng/mL to about 0.1 ng/mL, from about 16 ng/mL to about 0.1 ng/mL, from about 10 ng/mL to about 0.1 ng/mL, from about 8 ng/mL to about 0.1 ng/mL, from about 6 ng/mL to about 0.1 ng/mL, from about 4 ng/mL to about 0.1 ng/mL, from about 2 ng/mL to about 0.1 ng/mL, from about 1.8 ng/mL to about 0.1 ng/mL, from about 1.6 ng/mL to about 0.1 ng/mL, from about 1.4 ng/mL to about 0.1 ng/mL, from about 1.2 ng/mL to about 0.1 ng/mL, from about 1.0 ng/mL to about 0.1 ng/mL, from about 0.8 ng/mL to about 0.1 ng/mL, from about 0.6 ng/mL to about 0.1 ng/mL, from about 0.4 ng/mL to about 0.1 ng/mL, from about 0.2 ng/mL to about 0.1 ng/mL, and from about 0.16 ng/mL to about 0.1 ng/mL. In certain embodiments the VPBM has a value of diagnostic veterinary affinity value below about 0.1 ng/mL.

In certain embodiments, the VPBM is capable of diagnosing and/or providing prognosis for a GPD/C in a veterinary subject having a progastrin level at least about 0.1 times the normal progastrin level. Preferably the progastrin level of the veterinary subject is selected from the range: from about 0.1 times to about 500 times of the normal level; from about 0.25 times to about 500 times of the normal level; from about 0.5 times to about 500 times of the normal level; from about 0.75 times to about 500 times of the normal level; from about 1 times to about 500 times of the normal level; from about 2.5 times to about 500 times of the normal level; from about 5 times to about 500 times of the normal level; from about 7.5 times to about 500 times of the normal level; from about 10 times to about 500 times of the normal level; from about 12.5 times to about 500 times of the normal level; from about 15 times to about 500 times of the normal level; from about 17.5 times to about 500 times of the normal level; from about 20 times to about 500 times of the normal level; from about 20 times to about 500 times of the normal level; from about 25 times to about 500 times of the normal level; from about 30 times to about 500 times of the normal level; from about 35 times to about 500 times of the normal level; from about 40 times to about 500 times of the normal level; from about 45 times to about 500 times of the normal level; from about 50 times to about 500 times of the normal level; from about 55 times to about 500 times of the normal level; from about 60 times to about 500 times of the normal level; from about 65 times to about 500 times of the normal level; from about 70 times to about 500 times of the normal level; from about 75 times to about 500 times of the normal level; from about 80 times to about 500 times of the normal level; from about 85 times to about 500 times of the normal level; from about 90 times to about 500 times of the normal level; from about 95 times to about 500 times of the normal level; from about 100 times to about 500 times of the normal level; from about 125 times to about 500 times of the normal level; from about 150 times to about 500 times of the normal level; from about 175 times to about 500 times of the normal level; from about 200 times to about 500 times of the normal level; from about 250 times to about 500 times of the normal level; from about 300 times to about 500 times of the normal level; from about 350 times to about 500 times of the normal level; from about 400 times to about 500 times of the normal level; and from about 450 times to about 500 times of the normal level. In certain embodiments, the VPBM is capable of treating a GPD/C in a veterinary subject having a progastrin level exceeding 500 times the normal progastrin level.

In certain embodiments, the VPBM has an affinity constant to a dprogastrin-like protein (such as a dprogastrin-SUMO protein), the affinity constant selected from the range: from about 1×10⁻⁶ M to about 1×10⁻¹⁶ M, from about 5×10⁻⁷ M to about 1×10⁻¹⁶ M, from about 1×10⁻⁷M to about 1×10⁻¹⁶M, from about 5×10⁻⁸ M to about 1×10⁻¹⁶M, from about 1×10⁻⁸ M to about 1×10⁻¹⁶M, from about 5×10⁻⁹ M to about 1×10⁻¹⁶M, from about 1×10⁻⁹ M to about 1×10⁻¹⁶ M, from about 5×10⁻¹⁰ M to about 1×10⁻¹⁶ M, from about 1×10⁻¹⁰M to about 1×10⁻¹⁶M, from about 5×10⁻¹¹ M to about 1×10⁻¹⁶M, from about 1×10⁻¹¹ M to about 1×10⁻¹⁶ M, from about 5×10⁻¹² M to about 1×10⁻¹⁶ M, and from about 1×10⁻¹² to about 1×10⁻¹⁶ M.

In certain embodiments, two or more VPBM is used for diagnosis and/or prognosis in a veterinary subject. In certain embodiments, three or more VPBM are used for diagnosis and/or prognosis in a veterinary subject. In certain embodiments, three or more VPBM are used for diagnosis and/or prognosis in a veterinary subject. In certain embodiments, four or more VPBM are used for diagnosis and/or prognosis in a veterinary subject. In certain embodiments, a combination of a VPBM VPM N-terminus and a VPBM specific for the C-terminus are used for diagnosis and/or prognosis in a veterinary subject.

In certain embodiments, two pairs of a VPBM VPM N-terminus and a VPBM specific for the C-terminus are used for diagnosis and/or prognosis in a veterinary subject. In certain embodiments, the VPBM is a purified binding domain of a monoclonal antibody such as a heavy chain or a light chain, a purified monoclonal antibody, and/or combinations thereof. In certain embodiments, a system of VPBM is used for diagnosis and/or prognosis of a GPD/C in a veterinary subject. In certain embodiments, the method of diagnosing and/or providing prognosis for a GPD/C uses a VPBM system in a sandwich ELISA. In certain embodiments, the VPBM is conjugated to a detection agent, such as fluorophore.

In certain embodiments, the method of diagnosing and/or providing prognosis for a GPD/C comprising one or more of the following steps: absorbing a coating VPBM onto the surface of a well in an ELISA plate; removing unabsorbed coating VPBM; blocking the surface of the well in the ELISA plate with a blocking agent; removing unabsorbed blocking agent; adding a sample (such as a biological sample), which may contain a VPM, to the well in the ELISA plate; removing unbound sample; adding a detection VPBM to the well in the ELISA plate; detecting whether a VPM is present in the sample when a detection is bound to the VPM captured by the capturing VPBM. Detection of the VPM may be achieved by the disclosures herein. Exemplary blocking agents include bovine serum albumin and non-fat milk, among others.

In certain embodiments, the VPBM system is used in a multiplex ELISA. In certain embodiments the coating VPBM is a combination of one or more first VPBM and one or more second VPBM; and the detection VPBM is a combination of one or more first VPBM and one or more second VPBM. The coating VPBM and the detection VPBM can be the same.

In certain embodiments, the coating VPBM can be bound to a bead, such as a glass or magnetic bead, to detect a sample, such as a biological sample, which may contain a VPM. In certain embodiments, such as a western blot, the coating VPBM is used to precipitate any VPM from a sample, and the detection VPBM is used to detect the presence of any VPM. In certain embodiments, the VPBM system is able to detect a concentration of VPM at or above the threshold level for a GPD/C.

In certain embodiments, the VPBM is used for research and development purposes, including in an immuno assays, ELASA, radioimmuno assays, immunohistochemistry, immunofluorescence assays, and immuno-microscopy assays.

### X. Production of VPBM

In certain embodiments, the immunogens are immunogen 1 (SEQ ID NO: 1) and immunogen 2 (SEQ ID NO: 2), which each containing one or more sequences of amino acid not found in human progastrin (see SEQ ID NO: 5 and SEQ ID NO: 6) but present in a wide range of veterinary progastrin (see Table 12-Table 47). Thus,
Applicant has discovered that numerous amino acid residues and combinations of amino sequences at the N-terminus and C-terminus of veterinary progastrins are not present in human progastrin (see Table 12-Table 47).

US20220185035 describes monoclonal antibody raised against human progastrin, but not progastrin from dogs.

A specific exemplary antigenic region that can be used to construct an immunogen useful for obtaining N-terminal anti-PG monoclonal antibodies corresponds to residues 1 to 14 of hPG SWKPRSQQPDAPLG (SEQ ID NO: 25)) coupled to a linker sequence. Residues 55 to 80 of hPG QGPWLEEEEEAYGWMDFGRRSAEDEN Hybridomas.

### XI. Examples

### Example 1

### Production of VPBM

Healthy New Zealand White rabbits suitable for monoclonal antibody production are immunized with KLH conjugated to a VPM. Exemplary VPM include Peptide (i) and Peptide (ii) of Table 1 having a sequence of SEQ ID NO: 3 and SEQ ID NO: 4, respectively. Serum titers are measured with ELISA against 2 ug/mL of Peptide (i), 2 ug/mL Peptide (ii), and/or 2 ug/mL recombinant progastrin-His-SUMO.

### Example 2

### Sequencing of the VPM binding sites

A 96-well plate of individual antigen specific plasma cells are identified and isolated by FACS sorting. Antigen specific plasma cells are isolated to a 96-well plate as one cell per well. Heavy and light chains of antibody genes are amplified individually by single cell RT-PCR. Antibody heavy and light chains are assembled to expression plasmids and transiently expressed in HEK293 cells. Cell culture supernatants are assayed by ELISA against the peptides, as well as the progastrin-SUMO recombinant protein.

### Example 3

### Positive screening of VPBM

Antibody clones are selected based on the ELISA data from Peptide (i) and Peptide (ii). The dProg-1 clones or dProg-2 clones that reacted with the recombinant dProg-Sumo protein and either the Peptide (i) or Peptide (ii) are identified for deconvolution, expression, purification and biotinylation (see

### Example 4

### Negative screening of VPBM

Supernatants from 96-well plates described above can be screened in a similar ELISA as described above using human progastrin as a coating antigen at a concentration of 2 ug/mL in 1x PBS, 100 uL/well (see Table 48 to Table 54). Clones can be scored as positive for human progastrin if the ratio of optical density (OD) measurement (clone supernatant:anti-rabbit IgG) is greater than 1.0 and can be excluded from further analysis).

Quality and specificity of the monoclonal antibodies are also analysed in a sandwich ELISA (see Table 52-Table 54). Pairs of antibodies that bound VPM the best can be identified (see Table 52-Table 54).

### Example 5

### Production of heavy and light chains of monoclonal antibodies against VPM

Suitable vectors, such as the pRab293H2 vector, are used to clone the heavy chain of the monoclonal antibodies against VPM. Exemplary pRab293H2 vector and PCR inserts are shown in Figure 1. SEQ ID NO: 7 and SEQ ID NO: 8 respectively show exemplary sequences flanking the upstream and downstream of the PCR inserts containing the heavy chain of the monoclonal antibody against VPM.

Suitable vectors, such as the pRab293L3 vector, are used to clone the light chain of the monoclonal antibodies against VPM. Exemplary pRab293L3 vector and PCR inserts are shown in Figure 2. SEQ ID NO: 9 and SEQ ID NO: 10 respectively show exemplary sequences flanking the upstream and downstream of the PCR inserts containing the light chain of the monoclonal antibody against VPM.

### Example 6

### Production of chimeric multi-species antibodies against VPM

To use VPBM for therapeutic purposes without impacting their binding affinity to VPM, it can be useful to convert the VPBM into a chimeric form that is less immunogenic in a specific veterinary species. An exemplary construction of a chimeric antibody involves the sub-cloning and ligating of the heavy and light chain variable region genes into an exemplary vector such as pRab293 (SEQ ID NO: 9 and SEQ ID NO: 10), which contain the species-specific heavy- and light-chain immunoglobulin constant regions. Expression of the chimeric antibody is achieved by transfecting the vectors into a transfectable mammalian cell line, such as the Human Embryonic Kidney 293 cells (HEK293). The secreted antibody can be purified from the culture supernatant and tested in an ELISA to confirm the antigenbinding capacity after chimerization.

### Example 7

### Treating a veterinary subject with a VPBM

A veterinary subject can be treated with the VPBM. For example, a veterinary subject diagnosed with canine lymphoma can be treated with an amount of a neutralizing VPBM effective to provide a therapeutic benefit. The VPBM can be formulated in compositions with buffering agents and/or preservatives, and/or other excipients, with or without other therapeutic agents. Pharmaceutical compositions can be presented in unit dose forms containing a predetermined amount of VPBM. Such a unit can contain, for example, from about 1 mg to about 10 g. The VPBM can be administered as a singular injection or repeatedly via an intravenous route. Other routes of administration includes oral, transdermal, subcutaneous, intranasal, intramuscular, intraocular, topical, intrathecal, and intracerebroventricular, dependent upon the particular subject and type of cancer.

### Example 8

### Binding Specificity of VPBM

Binding specificity of the VPBM can be determined by assaying the VPBM against a VPM (such as a canine progastrin) using an ELISA assay essentially described below. Using a human progastrin or a progastrin specific to another veterinary species instead of the recombinant dog progastrin described below can further confirm cross-species specificity of the VPBM.

ELISA 96 well plates are coated with purified VPBM at a concentration of 2 ug/mL in 1x PBS, 100 uL/well. The wells are incubated at 4°C overnight or for 2 hours at room temperature. The plates are then washed 5x with 0.05% Tween-20 in PBS (washing buffer). Wells are blocked with 1% milk in 0.05% Tween-20 in PBS, 200 uL/well for 1 hour at room temperature. The plates are then washed 5x with washing buffer. 100 uL of dog progastrin His-SUMO protein is added to each well starting at (1: 1k) using a 3-fold serial dilutions in 0.1% BSA buffer. The mixtures are incubated for 1 hour at room temperature. Plates are then washed 5x with washing buffer. 100 uL of a second, purified VPBM (e.g., a dog progastrin antibody) can be used as a detection antibody. The detection antibody can be conjugated to biotin (1:5000) in 0.1% BSA. The detection antibody is added to each well and incubated for 1 hour at room temperature. The plates are washed 5x with washing buffer. 100 uL of streptavidin HRP (1:1000 dilution to a final concentration of 1-2 ug/mL in 0.1 BSA buffer) is added to each well and incubated at room temperature for 30 minutes. The plates are then washed 5x with the washing buffer. 50 uL/well of the ELISA substrate is added to each well and incubated for 10 minutes, followed by the addition of 50 uL of a stopping agent, 0.1N HCl. The plates are then read in a spectrophotometer at 450 nm.

### Example 9

### Binding Specificity of VPBM

Binding specificity of the VPBM can be determined by assaying the VPBM against a VPM (such as a canine progastrin) using an ELISA assay essentially described below. Using a human progastrin or a progastrin specific to another veterinary species instead of the recombinant dog progastrin described below can further confirm cross-species specificity of the VPBM.

ELISA 96 well plates are coated with purified VPBM at a concentration of 2 ug/mL in 1x PBS, 100 uL/well. The wells are incubated at 4°C overnight or for 2 hours at room temperature. The plates are then washed 5x with 0.05% Tween-20 in PBS (washing buffer). Wells are blocked with 1% milk in 0.05% Tween-20 in PBS, 200 uL/well for 1 hour at room temperature. The plates are then washed 5x with washing buffer. 100 uL of dog progastrin His-SUMO protein is added to each well starting at (1: 1k) using a 3-fold serial dilutions in 0.1% BSA buffer. The mixtures are incubated for 1 hour at room temperature. Plates are then washed 5x with washing buffer. 100 uL of a second, purified VPBM (e.g., a dog progastrin antibody) can be used as a detection antibody. The detection antibody can be conjugated to biotin (1:5000) in 0.1% BSA. The detection antibody is added to each well and incubated for 1 hour at room temperature. The plates are washed 5x with washing buffer. 100 uL of streptavidin HRP (1:1000 dilution to a final concentration of 1-2 ug/mL in 0.1 BSA buffer) is added to each well and incubated at room temperature for 30 minutes. The plates are then washed 5x with the washing buffer. 50 uL/well of the ELISA substrate is added to each well and incubated for 10 minutes, followed by the addition of 50 uL of a stopping agent, 0.1N HCl. The plates are then read in a spectrophotometer at 450 nm.

### Example 10

### VPBM is highly specific to veterinary species

The VPBM of this Application is highly specific to the veterinary species. When canine recombinant progastrin antibodies disclosed in this Application (both dProg-1-H2 mAb and dProg-2-A3 mAb) are used in a sandwich ELISA experiment, the canine recombination progastrin antibodies has strong (pg/mL range) binding affinity to canine recombinant progastrin, but does not bind to human recombinant progastrin (PG80).

Human Progastrin (PG80) control was obtained from Assay Genie, which was informed as P01350: 22-101aa sequence (not including the signal peptide). Each vial contained 500 pg of lyophilized material. One vial was brought up into solution with DI water to 2000 pg/mL and further diluted to 50 pg/mL.

4µg/mL 1-H2 Ab was coated overnight at 2-8°C. After overnight incubation, contents were removed and washed 1x with wash buffer. Block buffer was added to each well and incubated for 1 hour. After 1 hour incubation with block buffer, the contents were removed and washed 1x with wash buffer. Sample was then added to each well and incubated for 1 hour. After 1 hour incubation, the contents were removed and washed 3x with wash buffer. 0.5µg/mL biotinylated 2-A3 was added to each well and allowed to incubate for 2 hours. After 2 hour incubation, the contents were removed and washed 3x with wash buffer. 0.15 µg/mL streptavidin-HRP was then added to each well and allowed to incubate for 1 hour. After 1 hour incubation, the wells were washed 3x with wash buffer. To obtain OD readings, TMB substrate was added to each well and incubated for 15 minutes before addition of stop solution (0.16M H2SO4). Plate was then read with a plate reader.

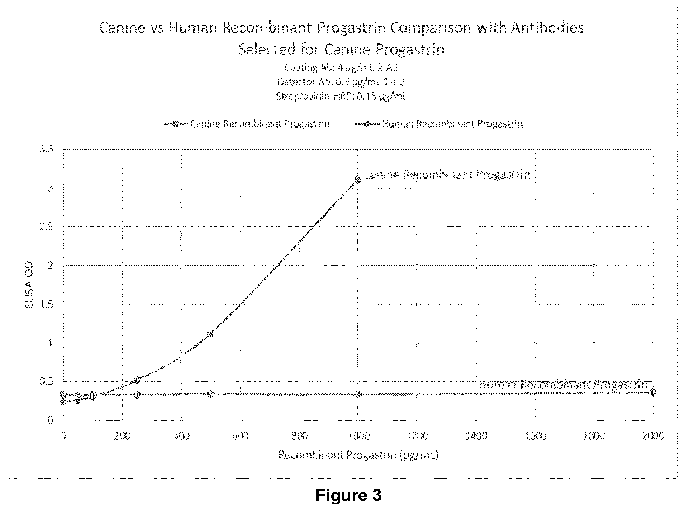

The results in FIG. 3 shows no observable signal when the human recombinant progastrin at up to 2000 pg/mL was added to the sandwich ELISA. In contrast, the canine recombinant progastrin has a strong dose response from 1000 to 100 pg/mL. These results show that canine recombinant progastrin antibodies (1-H2 and 2-A3) has strong specificity to canine progastrin but did not bind to the human recombinant progastrin.

### Example 11

### VPBM is highly specific to the veterinary molecule

The VPBM of this Application is highly specific to the veterinary molecule. When canine recombinant progastrin antibodies disclosed in this Application (both dProg-1-H2 mAb and dProg-2-A3 mAb) are used in a sandwich ELISA experiment, the canine recombination progastrin antibodies has strong (pg/mL range) binding affinity to canine recombinant progastrin, but does not bind to other canine proteins, such as canine gastrin.

Canine gastrin standard was obtained from the LS Bio's ELISA kit, which is used as standards for their ELISA protocol. Per the LS Bio's ELISA kit manual, 1 tube of standard was resuspended with 2mL of sample diluent to obtain the stock concentration of 1000 pg/mL. Further dilutions were completed to 50 pg/mL. 4µg/mL 1-H2 Ab was coated overnight at 2-8°C. After overnight incubation, contents were removed and washed 1x with wash buffer. Block buffer was added to each well and incubated for 1 hour. After 1 hour incubation with block buffer, the contents were removed and washed 1x with wash buffer. Sample was then added to each well and incubated for 1 hour. After 1 hour incubation, the contents were removed and washed 3x with wash buffer. 0.5µg/mL biotinylated 2-A3 was added to each well and allowed to incubate for 2 hours. After 2 hour incubation, the contents were removed and washed 3x with wash buffer. 0.15 µg/mL streptavidin-HRP was then added to each well and allowed to incubate for 1 hour. After 1 hour incubation, the wells were washed 3x with wash buffer. To obtain OD readings, TMB substrate was added to each well and incubated for 15 minutes before addition of stop solution (0.16M H2SO4). Plate was then read with a plate reader.

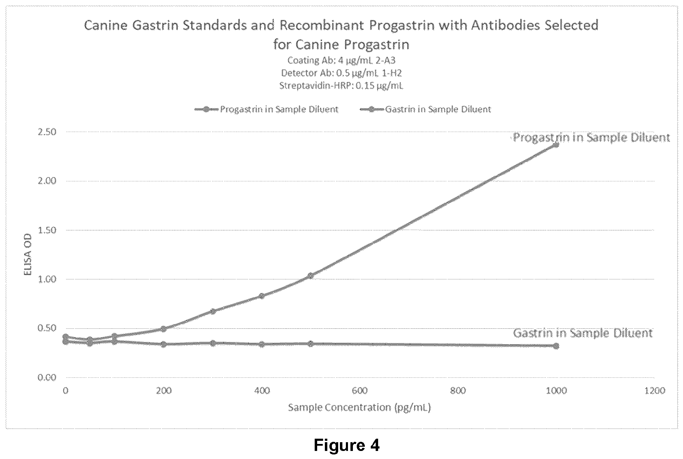

The results in FIG. 4 shows no dose response with the antibodies used in the progastrin ELISA. In contrast, the canine recombinant progastrin has a strong dose response from 1000 to 100 pg/mL. These results show that canine recombinant progastrin antibodies (1-H2 and 2-A3) has strong specificity to canine progastrin but did not bind to other canine proteins.

### XII. Tables

**Table 1. Residue sequence of progastrin-like immunogen.**

| Mammal | Progastrin -like molecule | SEQ ID NO. |
|---|---|---|
| Immunogen 1 | SWKPRS***RLQ***DAP***SGPG*** | SEQ ID NO: 1 |
| Immunogen 2 | QGPW***M***EEEE***A***AYGWMDFGRRSA***EEGDQRP*** | SEQ ID NO: 2 |
| Peptide (i) | SWKPRSRLQDAPSGPGC | SEQ ID NO: 3 |
| Peptide (ii) | CQGPWMEEEEAAYGWMDFGRRSAEEGDQRP | SEQ ID NO: 4 |
| hProGastrin(1-14) | SWKPRSQQPDAPLG | SEQ ID NO: 5 |
| hProGastrin(55-80) | QGPWLEEEEEAYGWMDFGRRSAEDEN | SEQ ID NO: 6 |

**Table 2. DNA sequences in exemplary vectors used to clone the heavy or light chain of the monoclonal antibody against the VPM.**

| Vector Name | Progastrin-like molecule | | SEQ ID NO. |
|---|---|---|---|
| pRab293H2 | ATGGAGACTGGGCTGCGCTGGCTTCT CCTGGTCGCTGTGCTC | Upstream of heavy chain PCR insert | SEQ ID NO: 7 |
| pRab293H2 | CACCCAGCTCCACGGTCACCCTGGGT TGTCTTGTGAAGGGA | Downstream of heavy chain PCR insert | SEQ ID NO: 8 |
| pRab293L4 | ATGGACACGAGGGCCCCCACTCAGCT GCTGGGGCTCCTGCTGCTCTGGCTCCC AGGTG | Upstream of light chain PCR insert | SEQ ID NO: 9 |
| pRab293L4 | TACTGTCCTCATCTTCCCACCAGCTGC TGATCAGGTGGCAACTGGAACAG | Downstream of light chain PCR insert | SEQ ID NO: 10 |

**Table 3. Residue sequence of veterinary progastrin protein.**

| Mammal | Progastrin-like molecule | SEQ ID NO. |
|---|---|---|
| Progastrin | SWKPRSRLQDAPSGPGANRGLEPHGLDQLGPASHHRRQLG LQGPPQLVADLSKKQGPWMEEEEAAYGWMDFGRRSAEEG DQRP | SEQ NO: 11 |

**Table 4. Exemplary DNA sequence encoding heavy chain binding domain for N terminus portions of VPM**

| PCR Insert | Binding domain against progastrin-like molecule | SEQ ID NO. |
|---|---|---|
| dProg1_B7HC1 / dProg1_D6HC1 | AAAGGTGTCCAGTGTCAGTCGCTGGAGGAGTCCGAGGGAGGT CTCTTCAAGCCAACGGATACCCTGACACTCACCTGCACAGTCT CTGGATTCTCCCTCATTAGCTATGGAGTGAGCTGGGTCCGCCA GGCTCCAGGGAAGGGGCTGGAATGGATCGGAGGCATTTGGCC TGGTGCTAGCACGTACTACGCGAGCTGGGCGAAAAGCCGATC CACCATCACCAGAAACACCAACCTGAACACGGTGACCCTGAA AATGACCAGTCTGACAGCCGCGGACACGGCCACCTATTTCTGT GCGAGGAATAGTGTTGGTTGGCTCTGGGGCCCAGGCACCCTGG TCACCGTCTCCTCAGGGCAACCTAAGGCTCCATCAGTCTTCCC ACTGGCCCCCTGCTGCGG | SEQ ID NO: 12 |
| dProg1_H2HC2 | AAAGGTGTCCAGTGTCAGTCGCTGGAGGAGTCCGGGGGTCGC CTGGTCAAGCCTGACGAAACCCTGACACTCACCTGCACAGTCT CTGGAATCGACCTCAGTAGCAATGCAATGAACTGGGTCCGCCA GGCTCCAGGGAACGGGCTGGAGTGGATCGGAGCCATTGATCG TGGTGGTGACACATACTACGCGAGCTGGGCGAGAAGCCGATC CACCATCACCAGAGACACCAACCTGAACACGGTGACTCTGAA AATGACCAGTCTGACAGCCGCGGACACGGCCACCTATTTCTGT GCGAGAGGTGCTTTTGCTTATCCTGAGGCTGGCATCTGGGGCC CAGGCACCCTGGTCACCGTCTCCTCAGGGCAACCTAAGGCTCC ATCAGTCTTCCCACTGGCCCCCTGCTGCGG | SEQ ID NO: 13 |

**Table 5. Exemplary DNA sequence encoding light chain binding domain for N terminus portions of VPM**

| PCR Insert | Binding domain against progastrin-like molecule | SEQ ID NO. |
|---|---|---|
| dProg1_B7LC10 / dProg1_D6LC1 | AGGTGCCACATTTGCCCAAGTGCTGACCCAGTCTGCATCGCCC GTGTCTGCAGCTGTGGGAGGCACAGTCACCATCAATTGCCAGG CCAGTCAGAGTATTTATAAGAACAACTACTTATCCTGGTATCA CCAGAAACCAGGGCAGCCTCCCAAGCTCCTGATCTATGATGCA TCCGATCTGGCATCTGGGGTCCCATCGCGGTTCAGTGGCAGTG GATCTGGGACACAGTTCACTCTCACCATCAGCGGCGTGCAGTG TGAAGATGCTGCCACTTACTACTGTCAAGGCGAATTTAGTTGT | SEQ ID NO: 14 |
| | AGTAGTGCTGATTGTTTTGCTTTCGGCGGAGGGACCGAGGTGG TGGTCAAAGGTGATCCAGTTGCACCTA | |
| dProg1_H2LC | AGGTGCCACATTTGCGCAAGTGCTGACCCAGACTCCAGCCTCC GTGTCTGCACCTGTGGGAGGCACAGTCACCATCAGTTGCCAGT CCAGTGAGAGTGTTTATAACAACAACTTAGCCTGGTTTCAGCA GAAACCAGGGCAGCCTCCCAAGCTCCTGATCTATGATGCATCC GATCTGGCATCTGGGGTCCCATCGCGGTTCAAGGGCAGTGGAT CTGGGACACAGTTCACTCTTACCATCAGCGACCTGGAGTGTGA CGATGCTGCCACTTACTACTGTGCAGGCGGTTATAGTGATGCT AATGCTTTCGGCGGAGGGACCGAGCTGGTGGTCAAAGGTGAT CCAGTTGCACCTA | SEQ ID NO: 15 |

**Table 6. Exemplary DNA sequence encoding heavy chain binding domain for C terminus portions of VPM**

| PCR Insert | Binding domain against VPM | SEQ ID NO. |
|---|---|---|
| dProg2_A3HC1 / dProg2_F4HC1 | AAAGGTGTCCAGTGTCAGTCGGTGGAGGAGTCCAGGGGAGGT CTCTTCAAGCCAACGGAGCCCCTGACACTCACCTGCACAGCCT CTGGATTCACCATCAGTAGCTACCACATGGGCTGGGTCCGCCA GACTCCAGGGAAGCAGCTGGAGTGGATCGGGTGGATTGGTAG TAGTGGTGCCACATACTACGCGAGCTGGGCGACAAGCCGGTC CACCATCACCAGAAACACCAACGACAACACGGTGACTCTGAA AATGACCAGTCTGACAGCCGCGGACACGGCCACCTATTTCTGT GCGAGAAGTGGTTGGGGTAGTTTTGACATCTGGGGCCCAGGC GCCCTGGTCACCGTCTCCTCAGGGCAACCTAAGGCTCCATCAG TCTTCCCACTGGCCCCCTGCTGCGG | SEQ ID NO: 16 |
| dProg2_D6HC1 | AAAGGTGTCCAGTGTCAGTCGGTGAAGGAGTCCGAGGGAGGT CTCTTCAAGCCAACGGAGCCCCTGACACTCACCTGCACAGTCT CTGGATTCTCCCTCAGTAGCGACAACATGGGCTGGGTCCGCCA GACTCCAGGGAAGCAGCTGGAGTGGATCGGGTGGATTGGTAG TAGTGGTAGCGCATACTACGCGAGGTGGGCGAAAAGCCGGTC CACCATCACCAGAAACACCAACGAGAACACGGTGACTCTGAA AATGACCAGTCTGACAGCCGCGGACACGGCCACCTATTTCTGT GCGAGAAGTGGTTGGGGTAGTTTTGACATATGGGGCCCAGGC ACCCTGGTCACCGTCTCCTCAGGGCAACCTAAGGCTCCATCAG TCTTCCCACTGGCCCCCTGCTGCGG | SEQ ID NO: 17 |

**Table 7. Exemplary DNA sequence encoding light chain binding domain for C terminus portions of VPM**

| PCR Insert | Binding domain against VPM | SEQ ID NO. |
|---|---|---|
| dProg2_A3LC5 / dProg2_F4LC1 | AGGTGCCACATTTGCGCAAGTGCTGACCCAGACTCCAGCCTCC GTGTCTGCCGCTGTGGGAGGCACAGTCACCATCAACTGCCAGT CCAGTCAGAGTGTTTATAATAACGACCGCTTAGCCTGGTTTCA GCAGAAACCAGGGCAGCCTCCCAAGCTCCTGATCTATCTGGCA TCCACTCTGGCATCTGGGGTCCCATCGCGGTTCAAAGGCAGTG GATCTGGGACACAGTTCACTCTCACAATCGGCGAAGTACAGTG TGACGATGCTGCCACTTACTACTGTCAAAGCTACTATAATGGT TATATTTTTGGTTTCGGCGGAGGGACCGAGGTGGTGGTCAAAG GTGATCCAGTTGCACCTA | SEQ ID NO: 18 |
| dProg2_D6LC3 | AGGTGCCACATTTGCGCAAGTGCTGACCCAGACTCCATCCCCC GTGTCTGCAGCTGTGGGAAGCACAGTCACCATCAGTTGCCAGT CCAGTCAGAGTGTTTATAGTAACAACCGCTTAGCCTGGTTTCA ACAGAAACCAGGGCAGCCTCCCAAGCTCCTGATCTATCTGGCA TCCACTCTGGCATCTGGGGTCCCATCGCGGTTCAAAGGCAGTG GATCTGGGACACAGTTCACTCTCACAATCAGCGAAGTACAGTG TGACGATGCTGCCACTTACTACTGTCAAGGCTATTATAGTGGT | SEQ ID NO: 19 |
| | TATATTTTTGGTTTCGGCGGAGGGACCGAGGTGGTAGTCAAAG GTGATCCAGTTGCACCTA | |

**Table 8. Portions of exemplary vector sequence encoding the heavy chain binding domain for N terminus portions of VPM**

| PCR Insert | Binding domain against VPM | SEQ ID NO. |
|---|---|---|
| dProg1_B7HC1 / dProg1_D6HC1 | ATGGAGACTGGGCTGCGCTGGCTTCTCCTGGTCGCTGTGCTCA AAGGTGTCCAGTGTCAGTCGCTGGAGGAGTCCGAGGGAGGTC TCTTCAAGCCAACGGATACCCTGACACTCACCTGCACAGTCTC TGGATTCTCCCTCATTAGCTATGGAGTGAGCTGGGTCCGCCAG GCTCCAGGGAAGGGGCTGGAATGGATCGGAGGCATTTGGCCT GGTGCTAGCACGTACTACGCGAGCTGGGCGAAAAGCCGATCC ACCATCACCAGAAACACCAACCTGAACACGGTGACCCTGAAA ATGACCAGTCTGACAGCCGCGGACACGGCCACCTATTTCTGTG CGAGGAATAGTGTTGGTTGGCTCTGGGGCCCAGGCACCCTGGT CACCGTCTCCTCAGGGCAACCTAAGGCTCCATCAGTCTTCCCA CTGGCCCCCTGCTGCGGGGACACACCCAGCTCCACGGTCACCC TGGGTTGTCTTGTGAAGGGATACCTCCCGGAACCCGTGACCGT GACCTGGAACTCCGGCACCCTGACCAATGGAGTGCGGACCTTC CCGAGCGTCAGGCAGTCCTCCGGGTTGTACAGCTTGTCTAGCG TGGTGTCCGTGACGTCGTCAAGCCAGCCTGTGACTTGCAATGT GGCCCATCCGGCCACCAACACCAAGGTCGACAAGACCGTGGC GCCTTCCACCTGTTCCAAGCCCACTTGCCCGCCGCCTGAGCTC CTGGGAGGACCGTCCGTGTTCATCTTCCCTCCAAAACCCAAGG ATACCCTGATGATTAGCCGCACTCCCGAAGTCACTTGCGTGGT CGTGGACGTGTCGCAGGACGATCCTGAGGTGCAGTTCACTTGG TACATCAACAACGAACAAGTCCGGACAGCTAGACCACCGCTG CGCGAGCAGCAGTTCAACTCAACTATCCGGGTGGTGTCCACCC TGCCGATCGCGCATCAGGATTGGCTGCGGGGGAAGGAGTTCA AGTGCAAAGTCCACAACAAGGCCCTGCCCGCCCCCATCGAAA AGACCATCTCCAAGGCTCGGGGCCAGCCTCTGGAGCCCAAAG TGTACACCATGGGCCCGCCTCGCGAGGAGCTCTCCTCACGCTC GGTGTCGCTGACTTGCATGATTAACGGCTTCTACCCTTCCGAC ATCTCCGTGGAATGGGAGAAGAACGGAAAAGCCGAAGATAAC TACAAGACCACGCCCGCCGTGCTGGACTCCGACGGAAGCTATT TCCTGTACTCCAAGCTCTCCGTCCCCACTTCGGAATGGCAGAG GGGGGACGTGTTCACTTGCTCCGTGATGCACGAGGCACTCCAC AACCACTACACCCAAAAGAGCATTTCGCGGTCACCTGGCAAGT AA | SEQ ID NO: 20 |
| dProg1_H2HC2 | ATGGAGACTGGGCTGCGCTGGCTTCTCCTGGTCGCTGTGCTCA AAGGTGTCCAGTGTCAGTCGCTGGAGGAGTCCGGGGGTCGCCT GGTCAAGCCTGACGAAACCCTGACACTCACCTGCACAGTCTCT GGAATCGACCTCAGTAGCAATGCAATGAACTGGGTCCGCCAG GCTCCAGGGAACGGGCTGGAGTGGATCGGAGCCATTGATCGT GGTGGTGACACATACTACGCGAGCTGGGCGAGAAGCCGATCC ACCATCACCAGAGACACCAACCTGAACACGGTGACTCTGAAA ATGACCAGTCTGACAGCCGCGGACACGGCCACCTATTTCTGTG CGAGAGGTGCTTTTGCTTATCCTGAGGCTGGCATCTGGGGCCC AGGCACCCTGGTCACCGTCTCCTCAGGGCAACCTAAGGCTCCA TCAGTCTTCCCACTGGCCCCCTGCTGCGGGGACACACCCAGCT CCACGGTCACCCTGGGTTGTCTTGTGAAGGGATACCTCCCGGA ACCCGTGACCGTGACCTGGAACTCCGGCACCCTGACCAATGGA GTGCGGACCTTCCCGAGCGTCAGGCAGTCCTCCGGGTTGTACA GCTTGTCTAGCGTGGTGTCCGTGACGTCGTCAAGCCAGCCTGT GACTTGCAATGTGGCCCATCCGGCCACCAACACCAAGGTCGAC AAGACCGTGGCGCCTTCCACCTGTTCCAAGCCCACTTGCCCGC CGCCTGAGCTCCTGGGAGGACCGTCCGTGTTCATCTTCCCTCC AAAACCCAAGGATACCCTGATGATTAGCCGCACTCCCGAAGTC | SEQ ID NO: 21 |
| | ACTTGCGTGGTCGTGGACGTGTCGCAGGACGATCCTGAGGTGC AGTTCACTTGGTACATCAACAACGAACAAGTCCGGACAGCTA GACCACCGCTGCGCGAGCAGCAGTTCAACTCAACTATCCGGGT GGTGTCCACCCTGCCGATCGCGCATCAGGATTGGCTGCGGGGG AAGGAGTTCAAGTGCAAAGTCCACAACAAGGCCCTGCCCGCC CCCATCGAAAAGACCATCTCCAAGGCTCGGGGCCAGCCTCTGG AGCCCAAAGTGTACACCATGGGCCCGCCTCGCGAGGAGCTCTC CTCACGCTCGGTGTCGCTGACTTGCATGATTAACGGCTTCTAC CCTTCCGACATCTCCGTGGAATGGGAGAAGAACGGAAAAGCC GAAGATAACTACAAGACCACGCCCGCCGTGCTGGACTCCGAC GGAAGCTATTTCCTGTACTCCAAGCTCTCCGTCCCCACTTCGG AATGGCAGAGGGGGGACGTGTTCACTTGCTCCGTGATGCACG AGGCACTCCACAACCACTACACCCAAAAGAGCATTTCGCGGTC ACCTGGCAAGTAATCTTCCCTCCAAAACCCAAGGATACCCTGA TGATTAGCCGCACTCCCGAAGTCACTTGCGTGGTCGTGGNCGT GTCGCNNNCGATCCTGAGGTGCAGTTCACTTGGTACATCAACA ACGAACAAGTCCNGGACAGCTAGACCAN | |

**Table 9. Portions of exemplary vector sequence encoding the light chain binding domain for N terminus portions of VPM**

| PCR Insert | Binding domain against VPM | SEQ ID NO. |
|---|---|---|
| dProg1_B7LC10 / dProg1_D6LC1 | ATGGACACGAGGGCCCCCACTCAGCTGCTGGGGCTCCTGCTGC TCTGGCTCCCAGGTGCCACATTTGCCCAAGTGCTGACCCAGTC TGCATCGCCCGTGTCTGCAGCTGTGGGAGGCACAGTCACCATC AATTGCCAGGCCAGTCAGAGTATTTATAAGAACAACTACTTAT CCTGGTATCACCAGAAACCAGGGCAGCCTCCCAAGCTCCTGAT CTATGATGCATCCGATCTGGCATCTGGGGTCCCATCGCGGTTC AGTGGCAGTGGATCTGGGACACAGTTCACTCTCACCATCAGCG GCGTGCAGTGTGAAGATGCTGCCACTTACTACTGTCAAGGCGA ATTTAGTTGTAGTAGTGCTGATTGTTTTGCTTTCGGCGGAGGG ACCGAGGTGGTGGTCAAAGGTGATCCAGTTGCACCTACTGTCC TCATCTTCCCACCAGCTGCTGATCAGGTGGCAACTGGAACAGT CACCATCGTGTGCGTGGCTAACAAGTACTTCCCGGACGTGACC GTGACCTGGGAAGTCGACGGAACCACTCAGACCACTGGTATC GAGAACAGCAAGACGCCCCAGAACTCCGCCGATTGTACTTAT AACCTGTCCTCCACACTGACCCTCACCTCGACCCAGTACAATT CCCACAAGGAGTACACTTGCAAAGTCACCCAGGGAACCACTT CAGTGGTGCAGAGCTTCAACCGGGGGGATTGCTGA | SEQ ID NO: 22 |
| dProg1_H2LC | ATGGACACGAGGGCCCCCACTCAGCTGCTGGGGCTCCTGCTGC TCTGGCTCCCAGGTGCCACATTTGCGCAAGTGCTGACCCAGAC TCCAGCCTCCGTGTCTGCACCTGTGGGAGGCACAGTCACCATC AGTTGCCAGTCCAGTGAGAGTGTTTATAACAACTTAGCCT GGTTTCAGCAGAAACCAGGGCAGCCTCCCAAGCTCCTGATCTA TGATGCATCCGATCTGGCATCTGGGGTCCCATCGCGGTTCAAG GGCAGTGGATCTGGGACACAGTTCACTCTTACCATCAGCGACC TGGAGTGTGACGATGCTGCCACTTACTACTGTGCAGGCGGTTA TAGTGATGCTAATGCTTTCGGCGGAGGGACCGAGCTGGTGGTC AAAGGTGATCCAGTTGCACCTACTGTCCTCATCTTCCCACCAG CTGCTGATCAGGTGGCAACTGGAACAGTCACCATCGTGTGCGT GGCTAACAAGTACTTCCCGGACGTGACCGTGACCTGGGAAGTC GACGGAACCACTCAGACCACTGGTATCGAGAACAGCAAGACG CCCCAGAACTCCGCCGATTGTACTTATAACCTGTCCTCCACAC TGACCCTCACCTCGACCCAGTACAATTCCCACAAGGAGTACAC TTGCAAAGTCACCCAGGGAACCACTTCAGTGGTGCAGAGCTTC AACCGGGGGGATTGCTGA | SEQ ID NO: 23 |

**Table 10. Portions of exemplary vector sequence encoding the heavy chain binding domain for C terminus portions of VPM**

| PCR Insert | Binding domain against VPM | SEQ ID NO. |
|---|---|---|
| dProg2_A3HC1 / dProg2_F4HC1 | ATGGAGACTGGGCTGCGCTGGCTTCTCCTGGTCGCTGTGCTCA AAGGTGTCCAGTGTCAGTCGGTGGAGGAGTCCAGGGGAGGTC TCTTCAAGCCAACGGAGCCCCTGACACTCACCTGCACAGCCTC TGGATTCACCATCAGTAGCTACCACATGGGCTGGGTCCGCCAG ACTCCAGGGAAGCAGCTGGAGTGGATCGGGTGGATTGGTAGT AGTGGTGCCACATACTACGCGAGCTGGGCGACAAGCCGGTCC ACCATCACCAGAAACACCAACGACAACACGGTGACTCTGAAA ATGACCAGTCTGACAGCCGCGGACACGGCCACCTATTTCTGTG CGAGAAGTGGTTGGGGTAGTTTTGACATCTGGGGCCCAGGCGC CCTGGTCACCGTCTCCTCAGGGCAACCTAAGGCTCCATCAGTC TTCCCACTGGCCCCCTGCTGCGGGGACACACCCAGCTCCACGG TCACCCTGGGTTGTCTTGTGAAGGGATACCTCCCGGAACCCGT GACCGTGACCTGGAACTCCGGCACCCTGACCAATGGAGTGCG GACCTTCCCGAGCGTCAGGCAGTCCTCCGGGTTGTACAGCTTG TCTAGCGTGGTGTCCGTGACGTCGTCAAGCCAGCCTGTGACTT GCAATGTGGCCCATCCGGCCACCAACACCAAGGTCGACAAGA CCGTGGCGCCTTCCACCTGTTCCAAGCCCACTTGCCCGCCGCC TGAGCTCCTGGGAGGACCGTCCGTGTTCATCTTCCCTCCAAAA CCCAAGGATACCCTGATGATTAGCCGCACTCCCGAAGTCACTT GCGTGGTCGTGGACGTGTCGCAGGACGATCCTGAGGTGCAGTT CACTTGGTACATCAACAACGAACAAGTCCGGACAGCTAGACC ACCGCTGCGCGAGCAGCAGTTCAACTCAACTATCCGGGTGGTG TCCACCCTGCCGATCGCGCATCAGGATTGGCTGCGGGGGAAG GAGTTCAAGTGCAAAGTCCACAACAAGGCCCTGCCCGCCCCC ATCGAAAAGACCATCTCCAAGGCTCGGGGCCAGCCTCTGGAG CCCAAAGTGTACACCATGGGCCCGCCTCGCGAGGAGCTCTCCT CACGCTCGGTGTCGCTGACTTGCATGATTAACGGCTTCTACCC TTCCGACATCTCCGTGGAATGGGAGAAGAACGGAAAAGCCGA AGATAACTACAAGACCACGCCCGCCGTGCTGGACTCCGACGG AAGCTATTTCCTGTACTCCAAGCTCTCCGTCCCCACTTCGGAAT GGCAGAGGGGGGACGTGTTCACTTGCTCCGTGATGCACGAGG CACTCCACAACCACTACACCCAAAAGAGCATTTCGCGGTCACC TGGCAAGTAA | SEQ ID NO: 24 |
| dProg2_D6HC1 | ATGGAGACTGGGCTGCGCTGGCTTCTCCTGGTCGCTGTGCTCA AAGGTGTCCAGTGTCAGTCGGTGAAGGAGTCCGAGGGAGGTC TCTTCAAGCCAACGGAGCCCCTGACACTCACCTGCACAGTCTC TGGATTCTCCCTCAGTAGCGACAACATGGGCTGGGTCCGCCAG ACTCCAGGGAAGCAGCTGGAGTGGATCGGGTGGATTGGTAGT AGTGGTAGCGCATACTACGCGAGGTGGGCGAAAAGCCGGTCC ACCATCACCAGAAACACCAACGAGAACACGGTGACTCTGAAA ATGACCAGTCTGACAGCCGCGGACACGGCCACCTATTTCTGTG CGAGAAGTGGTTGGGGTAGTTTTGACATATGGGGCCCAGGCA CCCTGGTCACCGTCTCCTCAGGGCAACCTAAGGCTCCATCAGT CTTCCCACTGGCCCCCTGCTGCGGGGACACACCCAGCTCCACG GTCACCCTGGGTTGTCTTGTGAAGGGATACCTCCCGGAACCCG TGACCGTGACCTGGAACTCCGGCACCCTGACCAATGGAGTGCG GACCTTCCCGAGCGTCAGGCAGTCCTCCGGGTTGTACAGCTTG TCTAGCGTGGTGTCCGTGACGTCGTCAAGCCAGCCTGTGACTT GCAATGTGGCCCATCCGGCCACCAACACCAAGGTCGACAAGA CCGTGGCGCCTTCCACCTGTTCCAAGCCCACTTGCCCGCCGCC TGAGCTCCTGGGAGGACCGTCCGTGTTCATCTTCCCTCCAAAA CCCAAGGATACCCTGATGATTAGCCGCACTCCCGAAGTCACTT GCGTGGTCGTGGACGTGTCGCAGGACGATCCTGAGGTGCAGTT CACTTGGTACATCAACAACGAACAAGTCCGGACAGCTAGACC ACCGCTGCGCGAGCAGCAGTTCAACTCAACTATCCGGGTGGTG TCCACCCTGCCGATCGCGCATCAGGATTGGCTGCGGGGGAAG | SEQ ID NO: 25 |
| | GAGTTCAAGTGCAAAGTCCACAACAAGGCCCTGCCCGCCCCC ATCGAAAAGACCATCTCCAAGGCTCGGGGCCAGCCTCTGGAG CCCAAAGTGTACACCATGGGCCCGCCTCGCGAGGAGCTCTCCT CACGCTCGGTGTCGCTGACTTGCATGATTAACGGCTTCTACCC TTCCGACATCTCCGTGGAATGGGAGAAGAACGGAAAAGCCGA AGATAACTACAAGACCACGCCCGCCGTGCTGGACTCCGACGG AAGCTATTTCCTGTACTCCAAGCTCTCCGTCCCCACTTCGGAAT GGCAGAGGGGGGACGTGTTCACTTGCTCCGTGATGCACGAGG CACTCCACAACCACTACACCCAAAAGAGCATTTCGCGGTCACC TGGCAAGTAA | |

**Table 11. Portions of exemplary vector sequence encoding the light chain binding domain for C terminus portions of VPM**

| PCR Insert | Binding domain against VPM | SEQ ID NO. |
|---|---|---|
| dProg2_A3LC5 / dProg2_F4LC1 | ATGGACACGAGGGCCCCCACTCAGCTGCTGGGGCTCCTGCTGC TCTGGCTCCCAGGTGCCACATTTGCGCAAGTGCTGACCCAGAC TCCAGCCTCCGTGTCTGCCGCTGTGGGAGGCACAGTCACCATC AACTGCCAGTCCAGTCAGAGTGTTTATAATAACGACCGCTTAG CCTGGTTTCAGCAGAAACCAGGGCAGCCTCCCAAGCTCCTGAT CTATCTGGCATCCACTCTGGCATCTGGGGTCCCATCGCGGTTC AAAGGCAGTGGATCTGGGACACAGTTCACTCTCACAATCGGC GAAGTACAGTGTGACGATGCTGCCACTTACTACTGTCAAAGCT ACTATAATGGTTATATTTTTGGTTTCGGCGGAGGGACCGAGGT GGTGGTCAAAGGTGATCCAGTTGCACCTACTGTCCTCATCTTC CCACCAGCTGCTGATCAGGTGGCAACTGGAACAGTCACCATCG TGTGCGTGGCTAACAAGTACTTCCCGGACGTGACCGTGACCTG GGAAGTCGACGGAACCACTCAGACCACTGGTATCGAGAACAG CAAGACGCCCCAGAACTCCGCCGATTGTACTTATAACCTGTCC TCCACACTGACCCTCACCTCGACCCAGTACAATTCCCACAAGG AGTACACTTGCAAAGTCACCCAGGGAACCACTTCAGTGGTGCA GAGCTTCAACCGGGGGGATTGCTGA | SEQ ID NO: 26 |
| dProg2_D6LC3 | ATGGACACGAGGGCCCCCACTCAGCTGCTGGGGCTCCTGCTGC TCTGGCTCCCAGGTGCCACATTTGCGCAAGTGCTGACCCAGAC TCCATCCCCCGTGTCTGCAGCTGTGGGAAGCACAGTCACCATC AGTTGCCAGTCCAGTCAGAGTGTTTATAGTAACAACCGCTTAG CCTGGTTTCAACAGAAACCAGGGCAGCCTCCCAAGCTCCTGAT CTATCTGGCATCCACTCTGGCATCTGGGGTCCCATCGCGGTTC AAAGGCAGTGGATCTGGGACACAGTTCACTCTCACAATCAGC GAAGTACAGTGTGACGATGCTGCCACTTACTACTGTCAAGGCT ATTATAGTGGTTATATTTTTGGTTTCGGCGGAGGGACCGAGGT GGTAGTCAAAGGTGATCCAGTTGCACCTACTGTCCTCATCTTC CCACCAGCTGCTGATCAGGTGGCAACTGGAACAGTCACCATCG TGTGCGTGGCTAACAAGTACTTCCCGGACGTGACCGTGACCTG GGAAGTCGACGGAACCACTCAGACCACTGGTATCGAGAACAG CAAGACGCCCCAGAACTCCGCCGATTGTACTTATAACCTGTCC TCCACACTGACCCTCACCTCGACCCAGTACAATTCCCACAAGG AGTACACTTGCAAAGTCACCCAGGGAACCACTTCAGTGGTGCA GAGCTTCAACCGGGGGGATTGCTGA | SEQ ID NO: 27 |

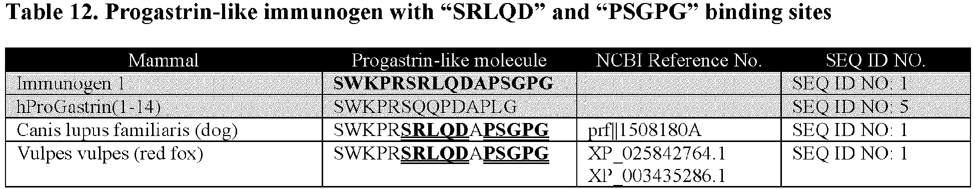

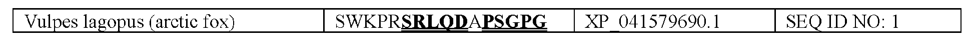

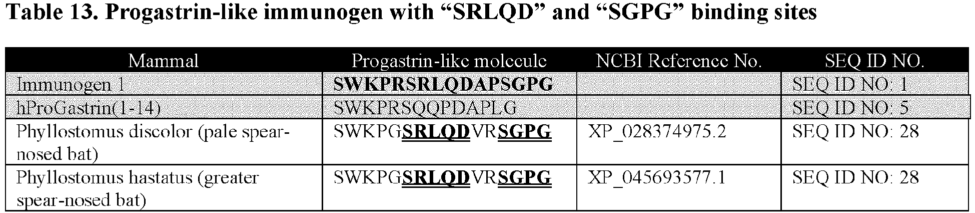

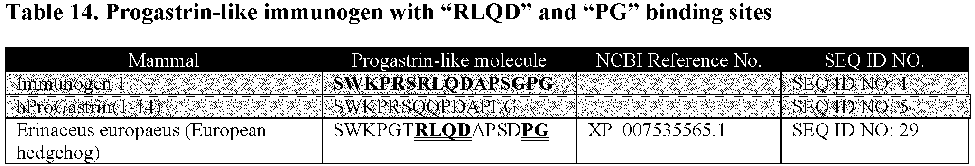

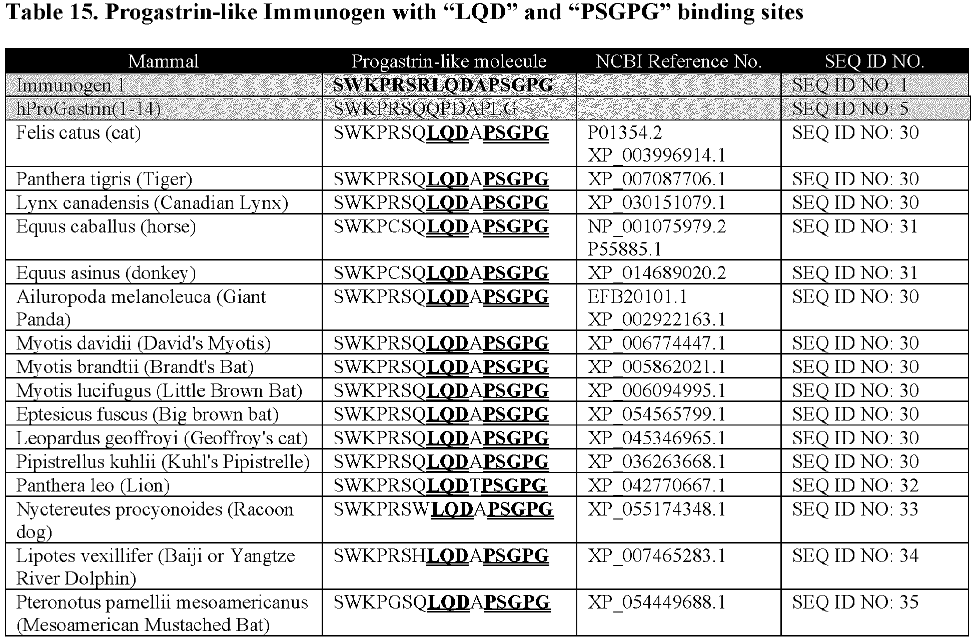

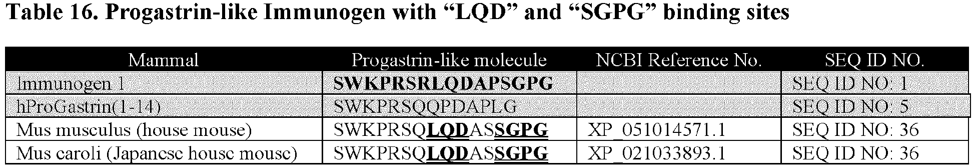

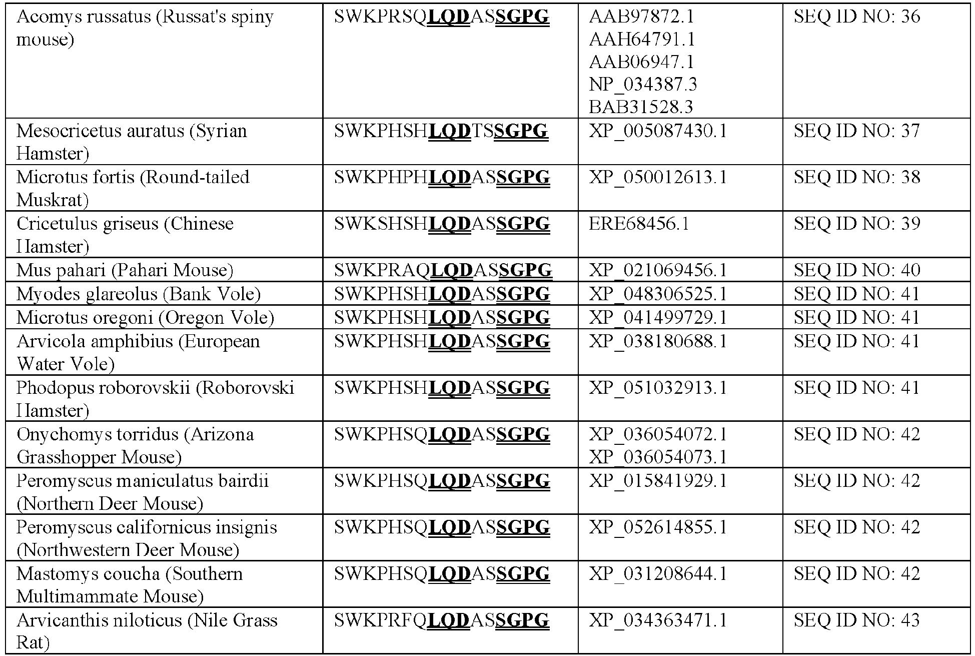

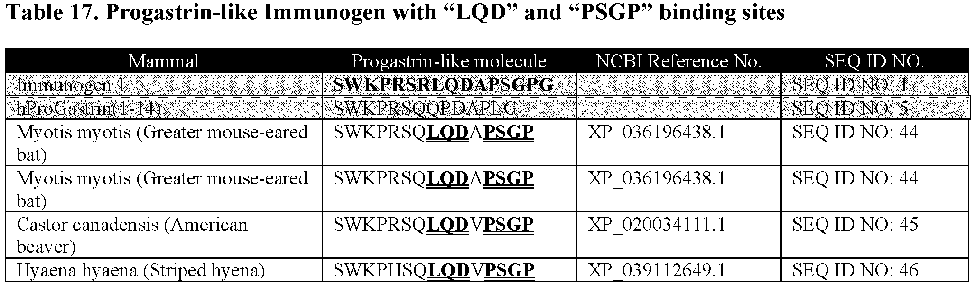

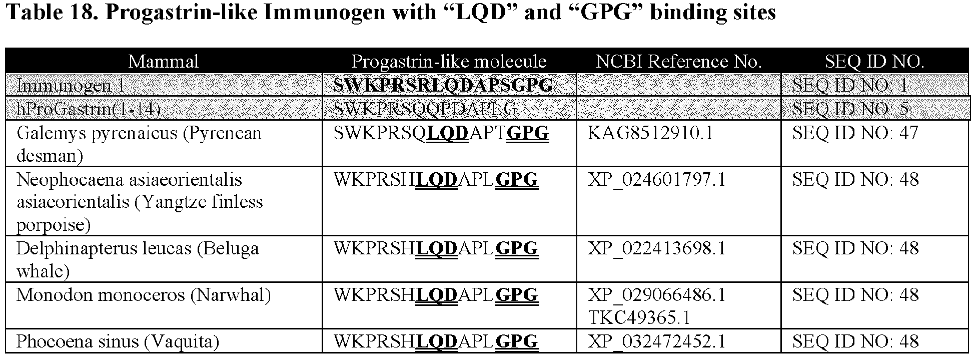

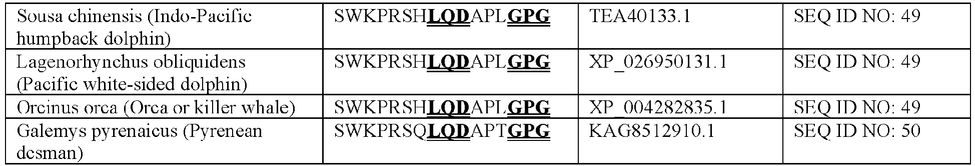

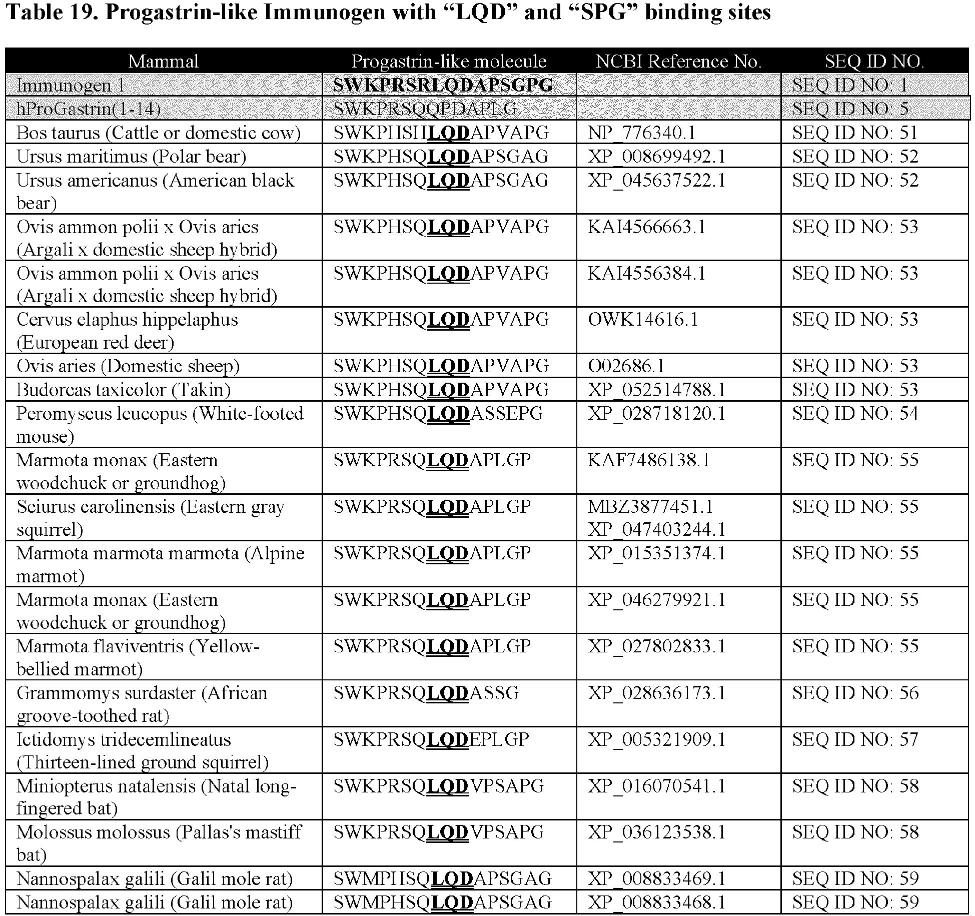

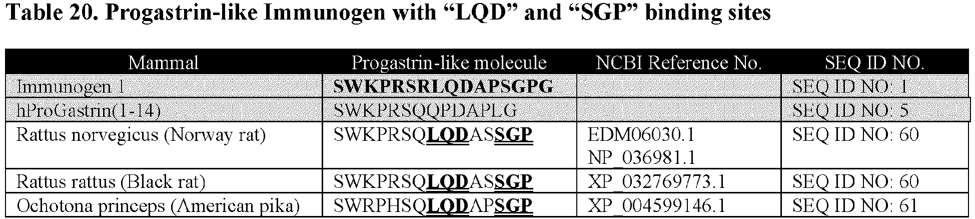

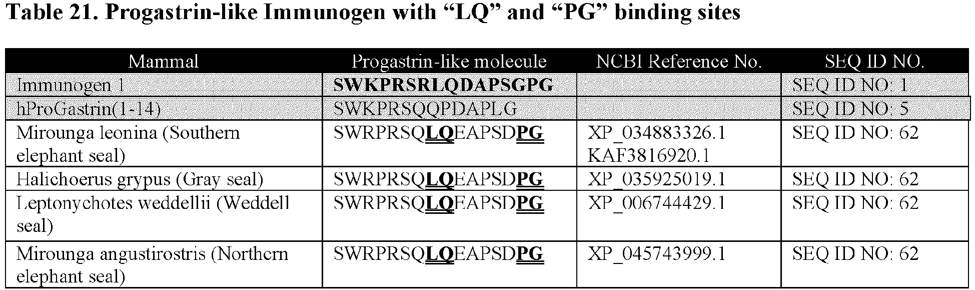

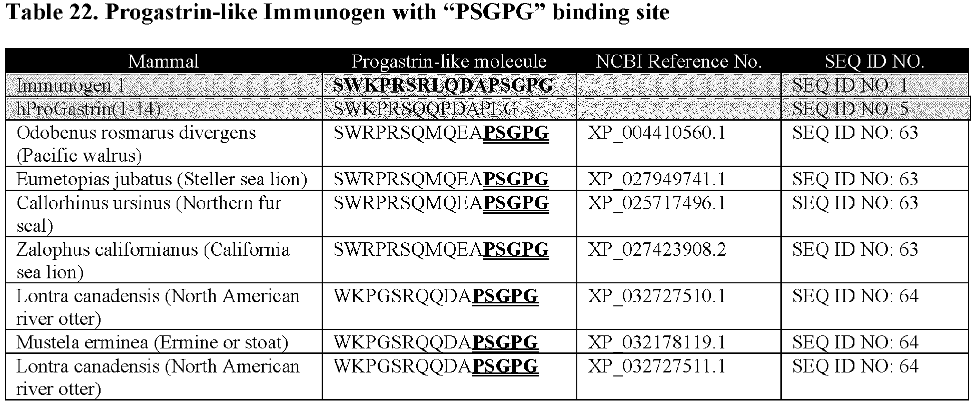

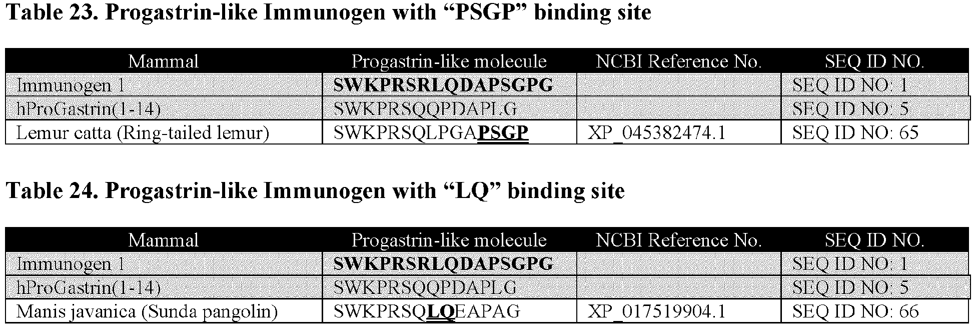

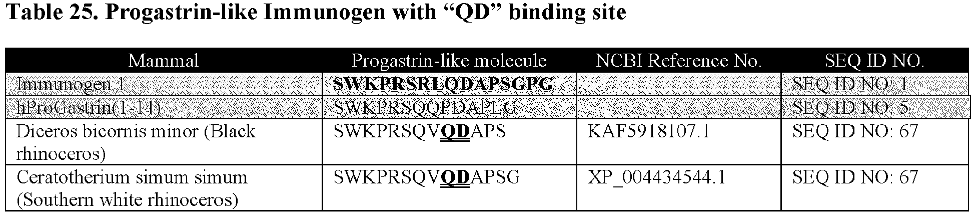

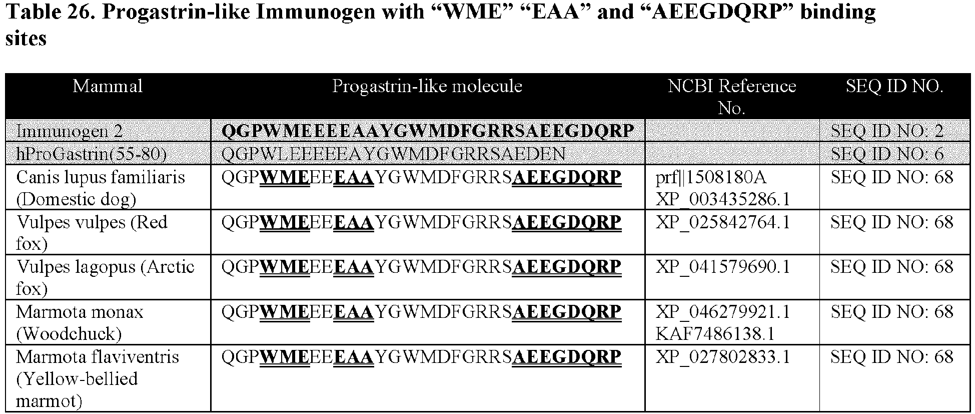

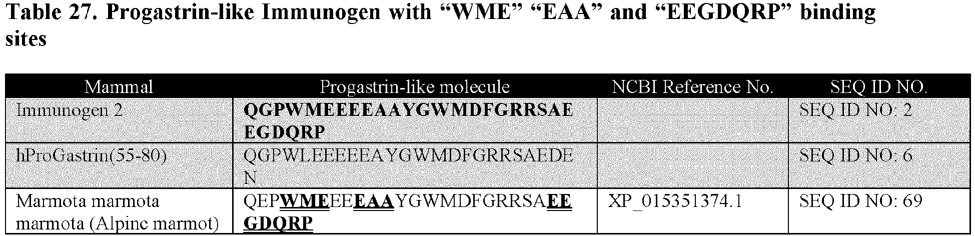

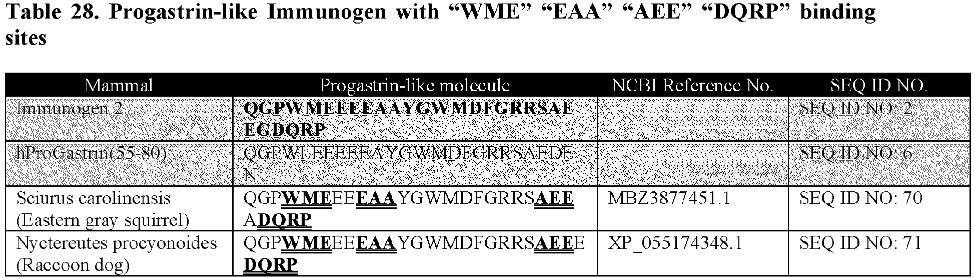

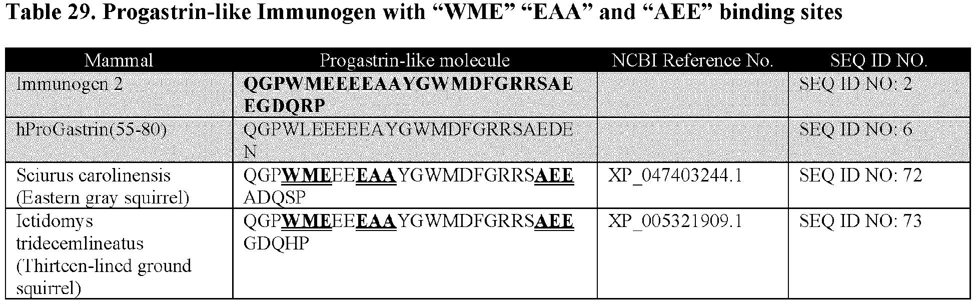

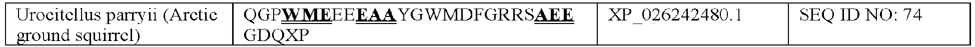

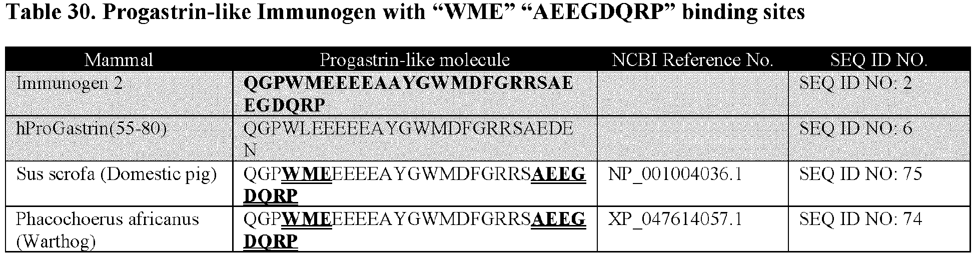

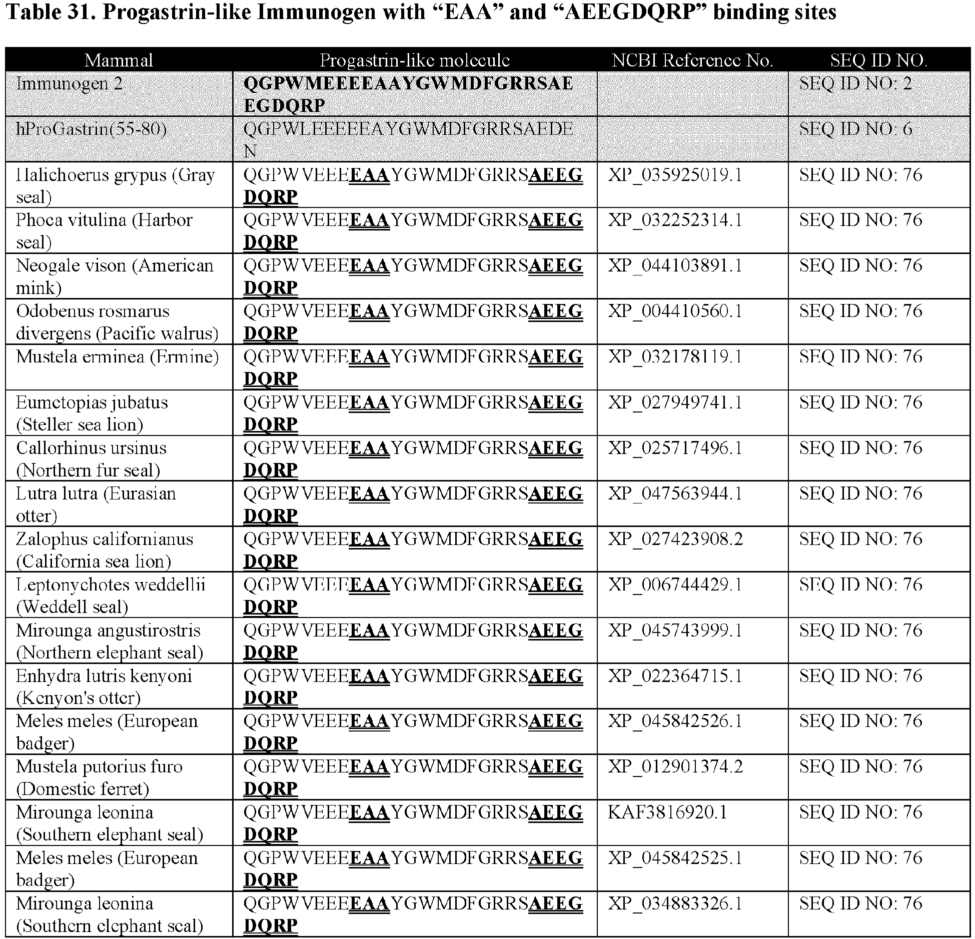

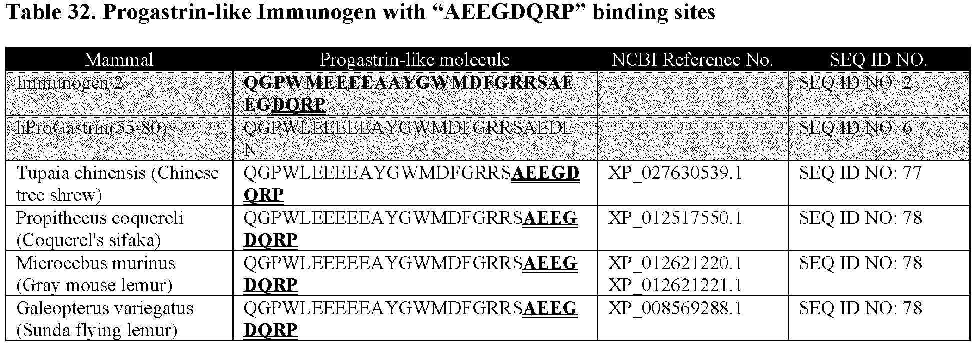

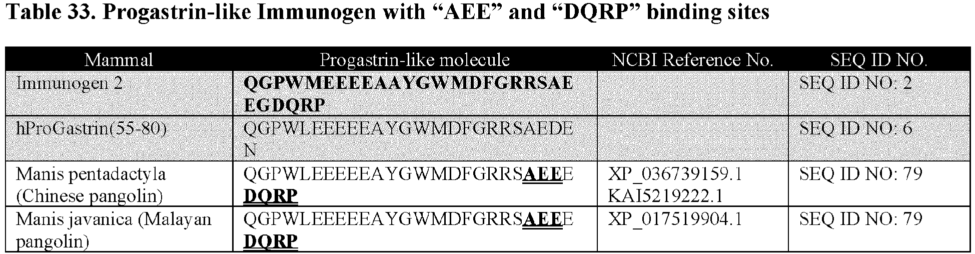

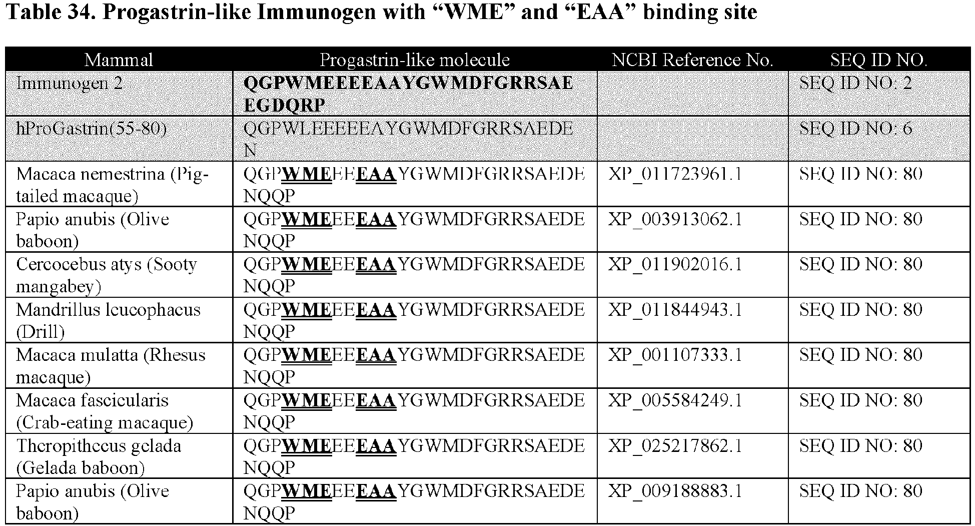

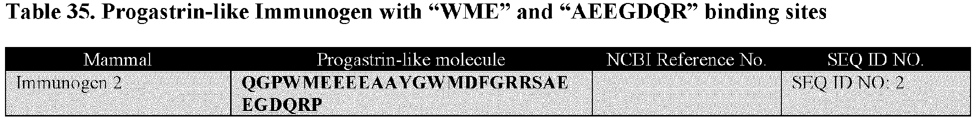

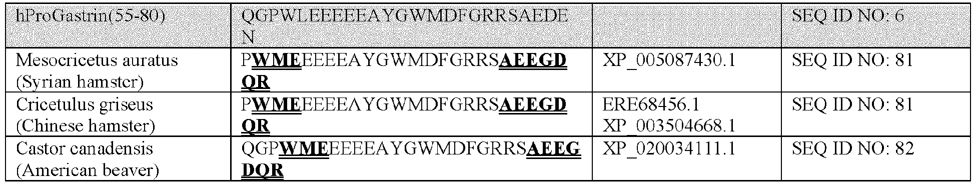

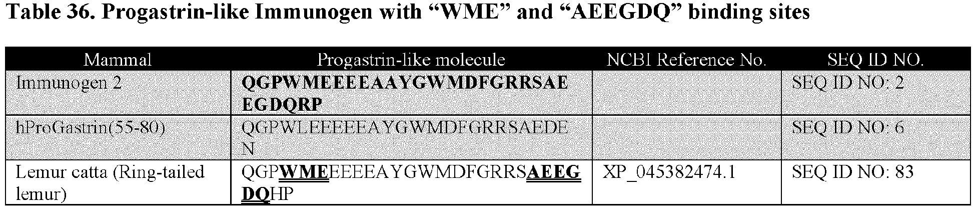

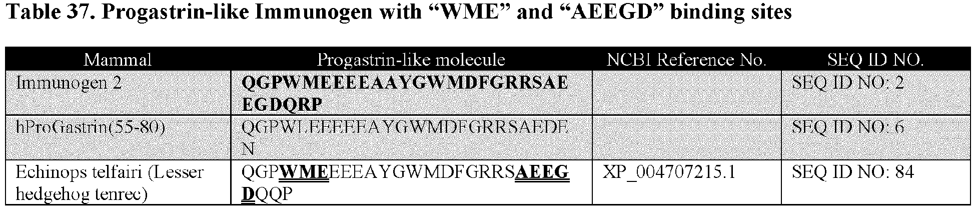

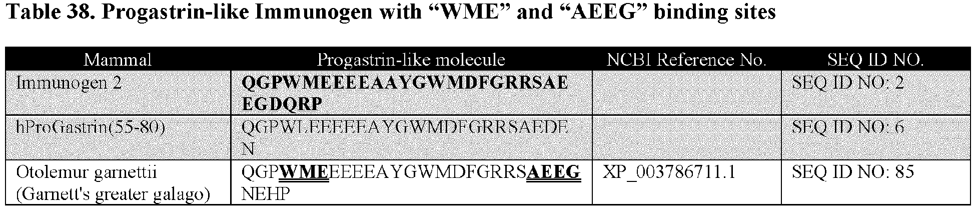

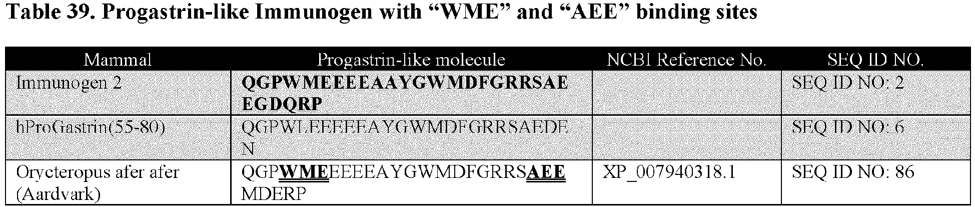

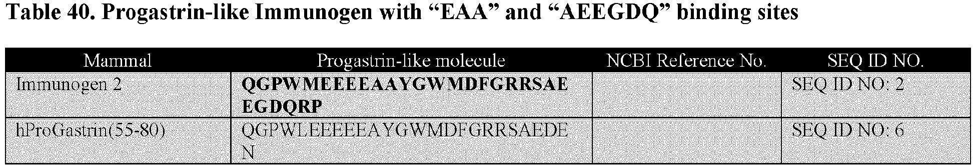

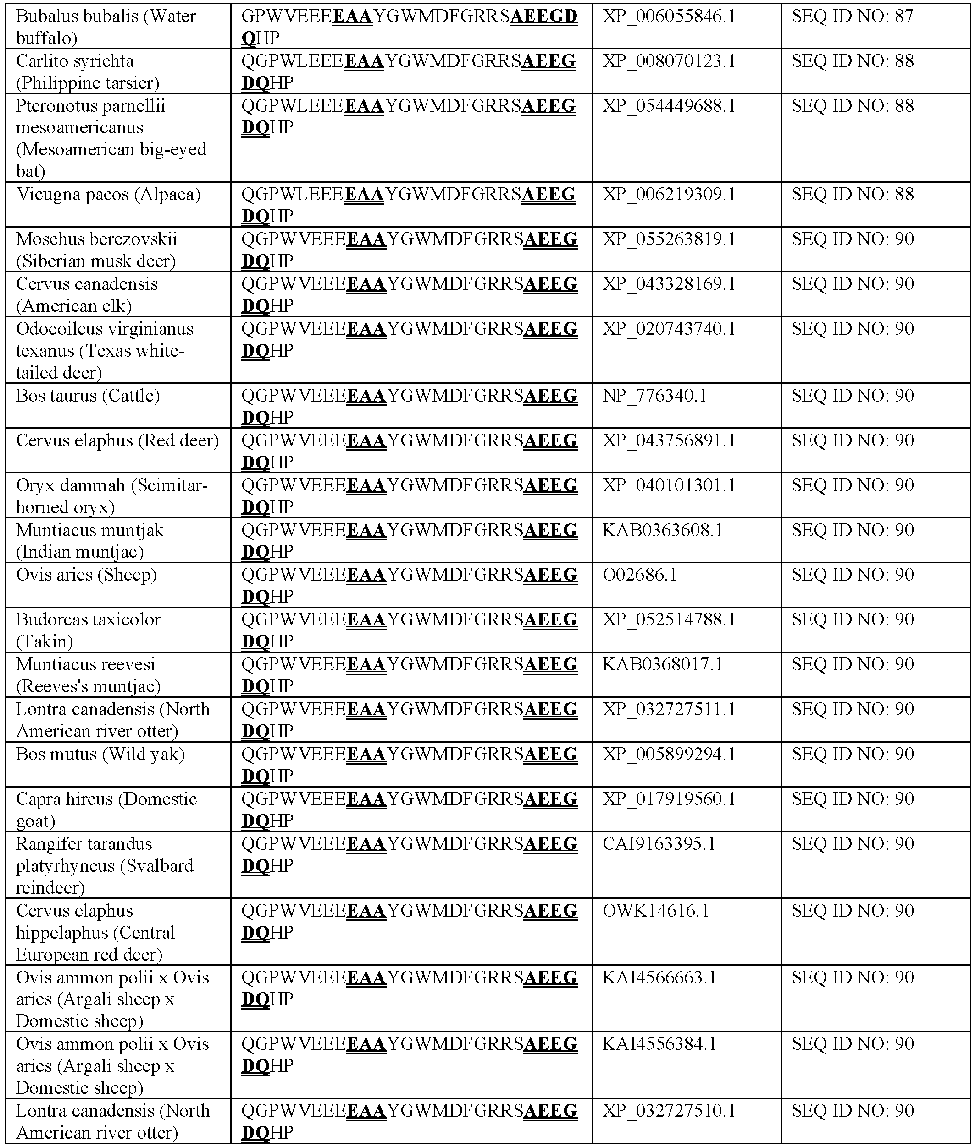

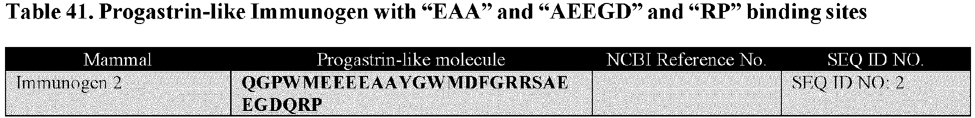

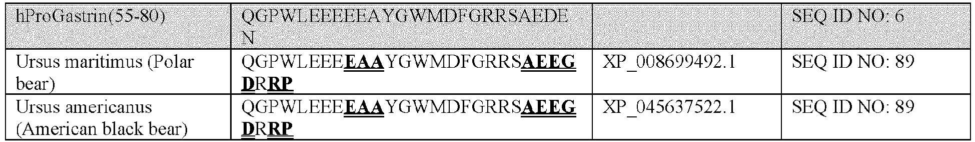

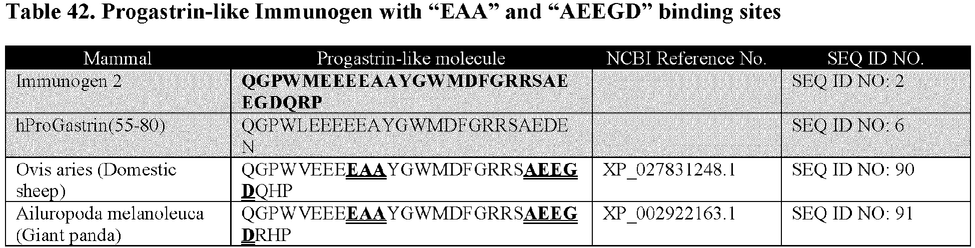

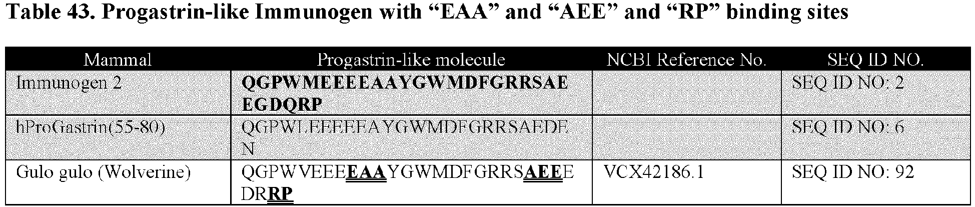

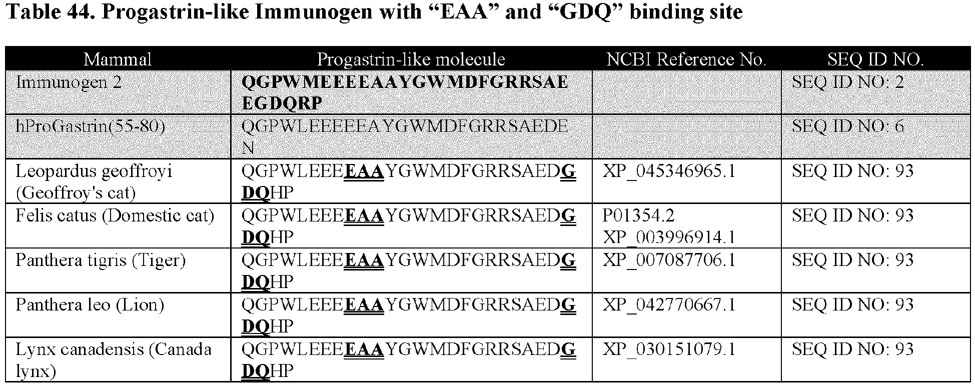

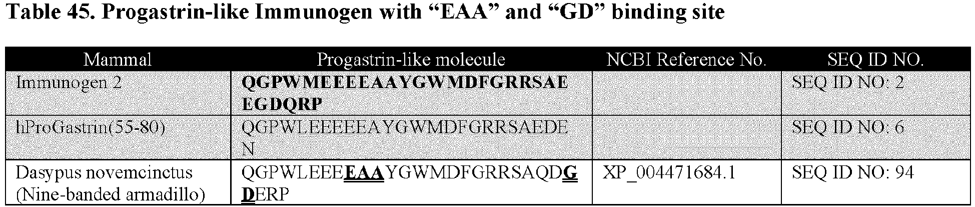

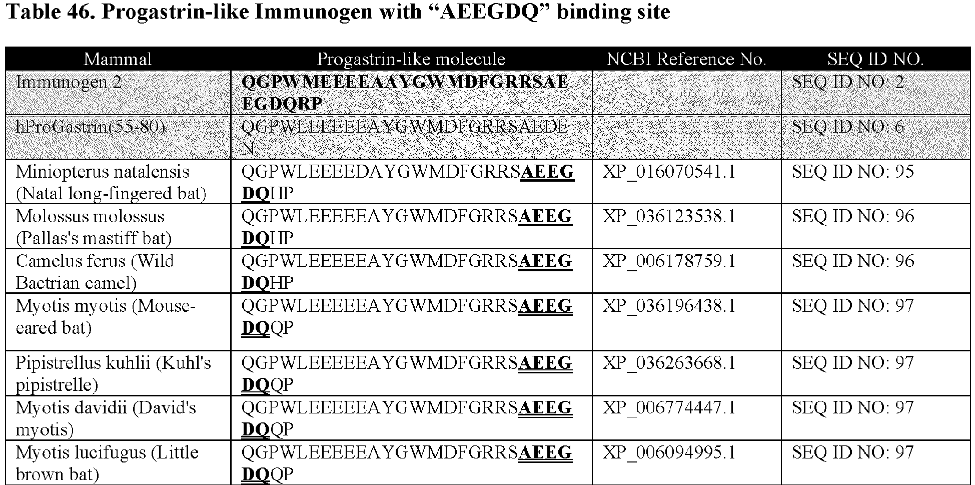

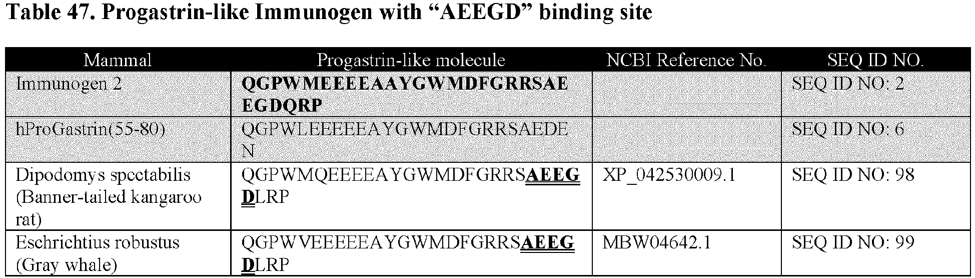

**Table 48. Progastrin-like Immunogen**

| Mammal | Progastrin-like molecule | SEQ ID NO. |
|---|---|---|
| VPS 1 | X₁**SRLQD**X₂**PSGPG** | SEQ ID NO: 100 |
| VPS 2 | X₃WMEX₄EAAX₅AEEGDQRP | SEQ ID NO: 101 |

**Table 49. dprogastrin-like protein**

| NAME | SEQUENCE | SEQ ID NO. |
|---|---|---|
| dprogastrin | SWKPRSRLQDAPSGPGANRGLEPHGLDQLGPASHHRRQLGI PQLVADLSKKQGPWMEEEEEAAYGWMDFGRRSA | SEQ ID NO: 102 |
| dProgastrin-SUMO protein | His-SUMO- SWKPRSRLQDAPSGPGANRGLEPHGLDQLGPASHHRRQLGI PQLVADLSKKQGPWMEEEEAAYGWMDFGRRSAEEGDQRP | SEQ ID NO: 103 |

**Table 50. Monocolonal antibodies with strong specificity against dProgastrin N terminus at low concentrations were selected.**

| | 1 | 2 | 3 | 4 | 5 | 6 | 7 | 8 |
|---|---|---|---|---|---|---|---|---|
| A | 1.1385; 0.2750 | 0.0336; 0.1885 | 0.0368; 0.3968 | 0.0497; 0.1478 | 0.0339; 0.1084 | 0.2989; 0.0578 | 0.0336; 0.1501 | 0.0412; 0.0540 |
| B | 0.0574; 0.1373 | 0.9262; 0.2521 | 0.0366; 0.3717 | 0.0493; 0.1735 | 0.0763; 0.2851 | 0.0384; 0.1694 | 0.8433; 0.1568 | 0.0344; 0.1078 |
| C | 0.0337; 0.2795 | 0.0361; 0.3019 | 0.0361; 0.2413 | 0.8922; 0.2120 | 0.0395; 0.1656 | 0.0481; 0.1230 | 0.0856; 0.1847 | 0.0347; 0.1142 |
| D | 0.0342; 0.1164 | 0.9047; 0.2059 | 0.0360; 0.2081 | 0.0361; 0.0971 | 0.0559; 0.1226 | 0.9285; 0.1855 | 0.0355; 0.1319 | - |
| E | 0.0338; 0.0773' | 0.0365; 0.3211' | 0.0359; 0.2394 | 0.0354; 0.2543 | 0.0345; 0.2675 | 0.0844; 0.3275 | 0.0387; 0.1486 | - |
| F | 1.1245; 0.2472 | 1.5581; 0.3706 | 0.0373; 0.2053 | 0.0349; 0.1616 | 0.0383; 0.2050 | 0.0334; 0.2843 | 0.0567; 0.0437' | - |
| G | 0.0333; 0.2774 | 1.1048; 0.2863 | 0.6105; 0.1233 | 0.0384; 0.1718 | 0.0346; 0.3528 | 0.0347; 0.3178 | 0.6113; 0.1269 | - |
| H | 0.0347; 0.3578' | 0.9992; 0.1761 | 0.0357; 0.2777 | 0.1315; 0.4183 | 0.0353; 0.3211 | 0.1184; 0.1578 | 0.0345; 0.9736 | - |
| Deep well ELISA reading of 59 different monoclonal antibodies against 2µg/ml dProgastrin- Sumo protein antigen versus 2µg/ml anti rabbit antibodies. Supernatants containing the antibodies were diluted at 1:2000. | | | | | | | | |
| Concentration standards are as follows: 2.1773, 125 ng/ml; 1.7899, 62.5 ng/ml; 1.3125, 31.25 ng/ml; 0.8913, 15.65 ng/ml; 0.4816, 7.81 ng/ml; 0.2428, 3.91 ng/ml; 0.1286, 1.95 ng/ml; 0.0357, NT. | | | | | | | | |
| Monoclonal antibodies with strong specificities against the dProgastrin N terminus at low concentrations include the H2 mAb, B7 mAb, and D6 mAb, among others. | | | | | | | | |

**Table 51. Monocolonal antibodies with strong specificity against dProgastrin C terminus at low concentrations were selected.**

| | 1 | 2 | 3 | 4 | 5 | 6 |
|---|---|---|---|---|---|---|
| A | 2.1300; | 1.3228; | 2.1878; | 1.1217; | 1.8966; | 1.6877; |
| | 1.3567 | 1.4329 | 1.5747 | 0.6219 | 1.2325 | 1.6104 |
| B | 1.8481; | 1.8896; | 2.0830; | 1.2943; | 1.4413; | 1.0088; |
| | 0.8396 | 1.5268 | 1.6334 | 1.5136 | 1.5896 | 1.1479 |
| C | 1.0885; | 1.4291; | 1.4224; | 2.1142; | 2.2925; | 1.7783; |
| | 1.3570 | 1.5262 | 1.3243 | 1.6974 | 1.7651 | 1.5448 |
| D | 0.9278; | 1.9507; | 0.9595; | 0.0671; | 1.8541; | 2.0195; |
| | 0.8692 | 1.0691 | 1.6828 | 1.4449 | 1.5819 | 1.5260 |
| E | 1.4596; | 1.9558; | 1.7645; | 0.8983; | 2.0711; | 1.9015; |
| | 1.1960 | 1.6021 | 1.6460 | 0.7975 | 1.7066 | 1.3970 |
| F | 1.6848; | 1.2666; | 1.9620; | 1.9854; | 1.6930; | 0.9823; |
| | 1.2731 | 1.6217 | 1.6803 | 1.6305 | 1.5572 | 1.2524 |
| G | 1.9417; | 1.8134; | 2.0003; | 1.7382; | 1.4222; | - |
| | 1.2678 | 1.3987 | 1.4961 | 1.2107 | 1.4056 | |
| H | 1.9122; | 2.1451; | 2.0387; | 2.2623; | 2.0249; | - |
| | 1.0243 | 1.4023 | 1.4699 | 1.4921 | 1.4217 | |
| Deep well ELISA reading of 46 different monoclonal antibodies against 2µg/ml dProgastrin-Sumo protein antigen versus 2µg/ml anti rabbit antibodies. Supernatants containing the antibodies were diluted at 1:2000. | | | | | | |
| Concentration standards are as follows: 1.916, 125 ng/ml; 1.7816, 62.5 ng/ml; 1.5376, 31.25 ng/ml; 1.0274, 15.65 ng/ml; 0.6652, 7.81 ng/ml; 0.3988, 3.91 ng/ml; 0.2317, 1.95 ng/ml; 0.0384, NT. | | | | | | |
| Monoclonal antibodies with strong specificities against the dProgastrin C terminus at low concentrations include the D6 mAb, A3 mAb, and F4 mAb, among others. | | | | | | |

**Table 52. dProgastrin Sandwich ELISA 1 showing the purified monoclonal antibodies have strong specificity to the respective dProgastrin regions on the dProgastrin-Sumo protein.**

| | 1 | 2 | 3 | 4 | 5 | 6 | 7 | 8 | 9 | 10 | Dilution¹ |
|---|---|---|---|---|---|---|---|---|---|---|---|
| A | 2.3101 | 2.3385 | 2.4104 | 2.1825 | 2.077 | 1.8578 | 1.8312 | 1.8841 | 1.803 | 1.5397 | 1:1K |
| B | 2.2645 | 2.0695 | 2.2393 | 2.1392 | 2.0411 | 1.2101 | 1 1.3199 | 1.1618 | 1.1848 | 1.3156 | 1:3K |
| C | 2.2208 | 2.1423 | 2.2646 | 2.0839 | 1.931 | 0.4301 | 0.5892 | 0.4175 | 0.5199 | 0.4352 | 1:9K |
| D | 2.1403 | 2.1868 | 2.1303 | 2.2698 | 1.924 | 0.2237 | 0.2355 | 0.184 | 0.1915 | 0.1542 | 11:27K |
| E | 2.1126 | 2.0007 | 2.2451 | 2.0459 | 1.7999 | 0.1128 | 0.1175 | 0.1089 | 0.1056 | 0.1102 | 1:81K |
| F | 1.7322 | 1.666 | 1.6472 | 1 1.6028 | 1.5073 | 0.0657 | 0.0716 | 0.0749 | 0.0749 | 0.0783 | 1:243K |
| G | 0.7995 | 0.8089 | 0.6133 | 0.5457 | 0.5202 | 0.0552 | 0.0531 | 0.056 | 0.0654 | 0.0677 | 1:729K |
| H | 0.0435 | 0.0412 | 0.0486 | 0.0465 | 0.0414 | 0.0448 | 0.0539 | 0.0512 | 0.062 | 0.0522 | NT |
| Detect² | dProg-2-A3 mAb | | dProg-2-D6 mAb | | dProg-2-F4 mAb | dProg-2-A3 mAb | | dProg-2-D6 mAb | | dProg-2-F4 mAb | |
| Coat³ | dProg-1_H2 | | | | | dprogastrin-1_B7 | | | | | |
| ^{1.} dProgastrin-Sumo protein at the indicated dilution factor, using stock solution at 0.5 mg/mL | | | | | | | | | | | |
| ² Indicated biotinylated detection antibody at 1:5K dilution, followed with detection with streptavidin HRP at 1:1K dilution. | | | | | | | | | | | |
| ^{3.} Indicated purified capturing antibody coating the ELISA plate at a concentration of 2µg/ml. | | | | | | | | | | | |

**Table 53. dProgastrin Sandwich ELISA 2 showing the purified monoclonal antibodies have strong specificity to the respective dProgastrin regions on the dProgastrin-Sumo protein.**

| | 1 | 2 | 3 | 4 | 5 | 6 | 7 | 8 | 9 | 10 | Diluation¹ |
|---|---|---|---|---|---|---|---|---|---|---|---|
| A | 1 2.2358 | 2.0459 | 2.116 | 1.8841 | 1.9135 | 2.6781 | 2.7292 | 2.5268 | 2.5716 | 2.1073 | 1:1K |
| B | 1.5295 | 1.6534 | 1.4183 | 1.4249 | 1.5186 | 3.1496 | 2.879 | 2.804 | 2.5875 | 2.1993 | 1:3K |
| C | 0.715 | 0.6928 | 0.6053 | 0.5073 | 0.7754 | 2.6711 | 2.8133 | 0.8121 | 0.6528 | 0.4287 | 1:9K |
| D | 0.2869 | 0.2616 | 0.2004 | 0.1903 | 0.2437 | 2.5046 | 2.2822 | 0.2049 | 0.201 | 0.1294 | 11:27K |
| E | 0.136 | 0.1195 | 0.1041 | 0.0996 | 0.1066 | 1.6919 | 1.7058 | 0.0723 | 0.0722 | 0.0673 | 1:81K |
| F | 0.0776 | 0.0725 | 0.076 | 0.0764 | 0.0648 | 0.596 | 0.6127 | 0.0588 | 0.0558 | 0.0539 | 1:243K |
| G | 0.056 | 0.0528 | 0.0585 | 0.0555 | 0.0581 | 0.1269 | 0.1289 | 0.0476 | 0.0483 | 0.0518 | 1:729K |
| H | 0.0478 | 0.0446 | 0.0581 | 0.049 | 0.0475 | 0.0519 | 0.0531 | 0.0487 | 0.0546 | 0.0506 | NT |
| Detect² | dProg-2-A3 mAb | | dProg-2-D6 mAb | | dProg-2-F4 mAb | dProg-1-H2 mAb | | dProg-1-B7 mAb | | dProg-1-D6 mAb | |
| Coat³ | dprogastrin-1_D6 mAb | | | | | dprogastrin-2_A3 mAb | | | | | |
| ^{1.} dProgastrin-Sumo protein at the indicated dilution factor, using a stock solution at 0.5 mg/mL | | | | | | | | | | | |
| ² Indicated biotinylated detection antibody at 1:5K dilution, followed with detection with streptavidin HRP at 1:1K dilution. | | | | | | | | | | | |
| ^{3.} Indicated purified capturing antibody coating the ELISA plate at a concentration of 2µg/ml. | | | | | | | | | | | |

**Table 54. dProgastrin Sandwich ELISA 3 showing the purified monoclonal antibodies have strong specificity to the respective dProgastrin regions on the dProgastrin-Sumo protein.**

| | 1 | 2 | 3 | 4 | 5 | 6 | 7 | 8 | 9 | 10 | Dilution¹ |
|---|---|---|---|---|---|---|---|---|---|---|---|
| A | 3.2643 | 3.193 | 1.7882 | 1.7388 | 1.4329 | 3.0882 | 2.9872 | 2.7477 | 2.2681 | 2.0586 | 1:1K |
| B | 3.2127 | 3.1268 | 1.2445 | 1.1809 | 0.9574 | 3.1738 | 3.138 | 2.6175 | 2.2021 | 2.0712 | 1:3K |
| C | 3.0829 | 3.0088 | 0.2972 | 0.3023 | 0.2822 | 3.2112 | 3.0351 | 0.8077 | 0.3097 | 0.2888 | 1:9K |
| D | 2.8108 | 2.6715 | 0.144 | 0.1411 | 0.144 | 2.7423 | 2.6639 | 0.1577 | 0.1462 | 0.0984 | 11:27K |
| E | 2.0104 | 1.874 | 0.084 | 0.0809 | 0.0895 | 1.9132 | 1.880 | 0.0627 | 0.0575 | 0.055 | 1:81K |
| F | 0.6818 | 0.6617 | 0.0674 | 0.0667 | 0.0605 | 0.5644 | 0.6133 | 0.0504 | 0.0478 | 0.0565 | 1:243K |
| G | 0.1668 | 0.1636 | 0.064 | 0.0633 | 0.0608 | 0.1327 | 0.1318 | 0.0447 | 0.041 | 0.0408 | 1:729K |
| H | 0.0554 | 0.053 | 0.0592 | 0.0582 | 0.0581 | 0.0422 | 0.0431 | 0.0417 | 0.0478 | 0.0396 | NT |
| Detect² | dProg-1-H2 mAb | | dProg-1-B7 mAb | | dProg-1-D6 mAb | dProg-1-H2 mAb | | dProg-1-B7 mAb | | dProg-1-D6 mAb | |
| Coat³ | dprogastrin-2_D6 | | | | | dprogastrin-2_F4 | | | | | |
| ^{1.} dProgastrin-Sumo protein at the indicated dilution factor, using stock solution at 0.5 mg/mL | | | | | | | | | | | |
| ² Indicated biotinylated detection antibody at 1:5K dilution, followed with detection with streptavidin HRP at 1:1K dilution. | | | | | | | | | | | |
| ^{3.} Indicated purified capturing antibody coating the ELISA plate at a concentration of 2µg/ml. | | | | | | | | | | | |

### XIII. References

The following publications, references, patents and patent applications are hereby incorporated by reference in their entireties.
Grimes S, Monoclonal antibodies to progastrin. U.S. Patent No. 8,158,128 B2.
Pannequin J, Boudier L, Joubert D, Hollande F. Monoclonal antibodies to progatrin and their uses. U.S. Published Patent Application No. 2022/0195035 A1.
Bonato C, Eng J, Hulmes JD, Miedel M, Pan YC, Yalow RS. Sequences of gastrins purified from a single antrum of dog and of goat. Peptides. 1986 Jul-Aug;7(4):689-93. doi: 10.1016/0196-9781(86)90045-8. PMID: 3763441.
Bardram L, Hilsted L, Rehfeld JF. Progastrin expression in mammalian pancreas. Proc Natl Acad Sci USA. 1990 Jan;87(l):298-302. Doi: 10.1073/pnas.87.1.298. PMID: 2296587; PMCID: PMC53250.
Paterson AC, Lockhart SM, Baker J, Neumann G, Baldwin GS, Shulkes A. Identity and regulation of stored and secreted progastrin-derived peptides in sheep. Endocrinology. 2004 Nov; 145(11):5129-40. doi: 10.1210/en.2004-0912. Epub 2004 Aug 12. PMID: 15308616.

## Claims

1. A veterinary progastrin binding molecule (VPBM) comprising a binding domain specific for a veterinary progastrin-like molecule (VPM),
wherein the VPM is derived from a mammal of the order consisting of the list selected from Carnivora, Rodentia, Chiroptera, Artiodactyla, Perissodactyla, Cetacea, and Eulipotyphla, and
wherein the VPBM is purified.

2. The VPBM of Claim 1, wherein the mammal belongs to the order Carnivora; and optionally or preferably, wherein the mammal belongs to the family Canidae.

3. The VPBM of Claim 1 or Claim 2, wherein mammal is a Canis lupus familiaris, a Felis catus, and Equus caballus.

4. The VPBM of Claim 1, Claim 2 or Claim 3, wherein mammal is:
(i) a Canis lupus familiaris; or
(ii) a Felis catus; or
(iii) a Equus caballus.

5. The VPBM of any one of the above claims, wherein the VPM is a veterinary progastrin, a veterinary gastrin, and/or combinations and/or portions thereof.

6. The VPBM of any one of the above claims, wherein the VPM is a recombinant molecule.

7. The VPBM of any one of the above claims, wherein the VPM is a recombinant protein comprise a progastrin and a SUMO protein and/or a progastrin-His and a SUMO protein.

8. The VPBM of any one of the above claims, wherein the VPM comprise the amino acid residue selected from the list consisting of:
SEQ ID Nos: 1-6, and 28-99
and/or combinations and/or portions thereof.

9. The VPBM of Claim 8, wherein:
(i) the VPM comprise the amino acid residue selected from the list consisting of:
SEQ ID Nos: 1-2, and 28-99
and/or combinations and/or portions thereof; or
(ii) the VPM comprise the amino acid residue selected from the list consisting of:
SEQ ID Nos: 1-2
and/or combinations and/or portions thereof.

10. The VPBM of any one of the above claims, wherein the molecule comprise a veterinary-specific progastrin sequence (VPS), wherein the VPS is either:
X₁SRLQDX₂PSGPGX₃ or
X₄WMEX₅EAAX₆AEEGDQRP,
wherein X₁, X₂, X₃, X₄, X₅, and X₆, is each a linker comprising between 0 to 40 residues, and
preferably the VPS comprise a sequence selected from the list consisting of SRLQD, PSGPG, SRLQD, SGPG, RLQD, PG, LQD, PSGP, GPG, SPG, SGP, LQ, QD, WME, EAA, AEEGDQRP, EEGDQRP, DQRP, AEE, DQRP, AEEGDQR, AEEGDQ, AEEGD, AEEG, RP, GDQ, and GD.

11. The VPBM of Claim 10,
wherein the VPS comprise one or more veterinary specific N-terminus amino acid residue selected from the list consisting of SRLQD, PSGPG, SRLQD, SGPG, RLQD, PG, LQD, PSGP, GPG, SPG, SGP, LQ, and QD; and/or
wherein the VPS comprise A list of veterinary specific C-terminus amino acid residue includes selected from the list consisting of WME, EAA, AEEGDQRP, EEGDQRP, DQRP, AEE, DQRP, AEEGDQR, AEEGDQ, AEEGD, AEEG, RP, GDQ, and GD; and/or
wherein the molecule is a monoclonal antibody.

12. The VPBM of any one of the above claims, wherein portions of the binding domain is encoded by a DNA sequence selected from the list consisting of SEQ ID NO: 12-27, and combinations thereof.

13. A composition comprising the VPBM of any of the above claim and a non-toxic excipient, carrier, and/or diluent.

14. An expression vector comprising a polynucleotide encoding the binding domain of any one of the above claims.

15. A method for treating a veterinary gastrin-promoted disease or condition (GPD/C) comprising administering to a veterinary subject a VPBM of any one of claims 1-12.
